# EUROPÄISCHE PATENTANMELDUNG

(11) **EP 1 671 654 A2**
(43) Veröffentlichungstag der Anmeldung: **21.06.2006**
(21) Anmeldenummer: 06002435.3
(22) Anmeldetag: 15.03.2002
(51) Int. Cl.: A61K 47/48

(54) **Hydroxyalkylstärke-Wirkstoff-Konjugate**

(30) Priorität: 16.03.2001 DE 10112825
(62) Teilanmeldung aus: 02742858.0
(71) Anmelder: Fresenius Kabi Deutschland GmbH, 61352 Bad Homburg v.d.H. (DE)
(72) Erfinder: Sommermeyer, Klaus, 61191 Rosbach (DE); Eichner, Wolfram, 35510 Butzbach (DE); Frie, Sven, 1967 Bramois (CH); Jungheinrich, Cornelius, 61350 Bad Homburg (DE); Scharpf, Roland, 63691 Ranstadt (DE); Lutterbeck, Katharina, 61169 Friedberg (DE)
(74) Vertreter: Wichmann, Hendrik

(57) **Zusammenfassung**

Die vorliegende Erfindung betrifft Verbindungen, die ein Konjugat aus Hydroxalkystärke (HAS) und einem Wirkstoff umfassen, wobei die Hydroxalkystärke entweder unmittelbar oder über einen Linker kovalent an den Wirkstoff gebunden ist. Die Erfindung betrifft ferner Verfahren zur Herstellung eines kovalenten HAS-Wirkstoff-Konjugates, bei denen man HAS und einen Wirkstoff in einem Reaktionsmedium miteinander umsetzt, dadurch gekennzeichnet, dass das Reaktionsmedium Wasser oder ein Gemisch von Wasser mit organischen Lösungsmittel, welches mindestens 10 Gew.-% Wasser umfasst, ist.

## Beschreibung

Die vorliegende Erfindung betrifft Verbindungen, die ein Konjugat aus einer Hydroxyalkylstärke (HAS) und einem Wirkstoff umfassen, wobei die Hydroxyalkylstärke entweder unmittelbar oder über einen Linker kovalent an den Wirkstoff gebunden ist. Die Erfindung betrifft ferner Verfahren zur Herstellung eines kovalenten HAS-Wirkstoff-Konjugates, bei denen man HAS und einen Wirkstoff in einem Reaktionsmedium miteinander umsetzt, dadurch gekennzeichnet, dass das Reaktionsmedium Wasser oder ein Gemisch von Wasser mit organischem Lösungsmittel, welches mindestens 10 Gew.-% Wasser umfaßt, ist. Die Erfindung betrifft auch die medizinische Verwendung der Konjugate.

### TECHNISCHER HINTERGRUND

Der klinische Einsatz vieler Pharma-Wirkstoffe wird durch eine Reihe von Problemen beeinträchtigt (vgl. Delgado et al., Critical Reviews in Therapeutic Drug Carrier Systems, Vol. 9 (3, 4), (1992) S. 249-304). Parenteral verabreichte native Proteine unterliegen beispielsweise der Ausscheidung durch das retikuloendotheliale System, die Leber, die Niere und die Milz. Die Ausscheidung erfolgt dabei in Abhängigkeit der Ladung der Kohlenhydratketten, der Präsenz zellulärer Rezeptoren für das Protein und von der Molekülform und -größe. Die Ausschlussgrenze der Glomerularfiltration der Niere liegt beispielsweise bei etwa 67 kD.

Als Folge proteolytischer Degradation ist ferner ein schneller Verlust der biologischen Aktivität zu beobachten.

Bakteriell exprimierte Proteine sowie andere rekombinante Proteine können eine erhöhte Immunogenität aufweisen und lebensbedrohliche Hypersensitivitätsreaktionen provozieren. Entsprechende Reaktionen verhindern natürlich die medizinische Verwendung dieser Produkte.

Aus diesem Grund wurde im Stand der Technik bereits seit Ende der 70er Jahre systematisch an der Verbesserung der Eigenschaften exogener Proteine durch chemische Modifikation, insbesondere Polymerisation oder Kopplung an makromolekulare Polymere geforscht. Viele Arbeiten konzentrierten sich dabei auf die Herstellung von Konjugaten aus Proteinen oder anderen Wirkstoffen einerseits und Polyethylenglykol (PEG) andererseits (vgl. US 4,179,337). Die Vorteile, die man sich von diesen Kopplungsreaktionen erhoffte, umfassen verbesserte in vivo Halbwertszeit der Proteine, verringerte Toxizität, verbesserte Stabilität und verbesserte Löslichkeit der Wirkstoffe (Abuchowski und Davis, Enzymes as drugs, Holcenberg und Rubberts, Herausgeber, S. 367-383, John Wiley & Sons N.Y. (1981).

Das Verfahren zur Kopplung der Wirkstoffe erwies sich jedoch als problematisch, da die aktive Gruppe des Proteins durch Kopplung an PEG inaktiviert wurde oder die Reaktionen das Reaktionsprodukt nicht in brauchbarer Ausbeute lieferten. Um eine spezifische Bindung zu erzielen, die die Aktivität des Wirkstoffs nicht beeinträchtigt, wurden aktive Gruppen in PEG oder den Wirkstoff eingeführt oder die Verbindungen mit einem Linker aneinander gebunden. Üblicherweise wird PEG dafür mit einer aktiven Gruppe versehen, welche nachfolgend kovalent an die kopplungsfähige Gruppe eines Proteins gebunden wird.

So wurde beispielsweise der Verlust der Bindungsaktivität von Antikörpern und deren Fragmenten nach deren Kopplung an PEG beschrieben (Kitamura et al., Cancer Res., Vol. 51 (1991), S. 4310-4315; und Pedley, et al., Br. J. Cancer, Vol. 79 (1994), S. 1126-1130). Zur Lösung dieses Problems schlagen Chapman et al. (Nature Biotech., Vol. 17 (1999), S. 780-783) vor, PEG an bestimmte Bindungsstellen des Antikörpers zu binden.

Der Aktivitätsverlust des Kopplungspartners wird ferner in WO 95/13090 beschrieben. Zur Lösung wird vorgeschlagen, PEG mit einer reaktiven Gruppe zu aktivieren und PEG über diese reaktive Gruppe in Gegenwart eines Tensides an α-Interferon zu binden. Als bevorzugte reaktive Gruppe wird N-Succinimid-Carbonat genannt, das unter den genannten Bedingungen mit der ε-Aminogruppe des Lysin eine Urethan-Bindung ausbilden soll.

Auch die WO 96/41813 offenbart Verfahren zur Herstellung eines Polymer-Polypeptid-Konjugates, bei denen das Polymer (insbesondere PEG) an einer spezifischen Stelle derivatisiert und anschließend an ein Polypeptid gebunden wird. Vorzugsweise wird dabei eine Amino-Oxi-Acetyl-Gruppe in PEG eingebracht und diese Verbindung anschließend an ein Polypeptid, insbesondere an IL-8, hG-CSF und IL-1 gebunden.

In der Literatur finden sich somit eine Vielzahl von Beispielen für entsprechende Konjugate; vgl. PEG-Insulin-Konjugate in US 4,179,337, bovine PEG-Hämoglobin-Konjugate in US 4,412,989, PEG-Ribonuklease-Konjugate und PEG-Superoxiddismutase-Konjugate in Veronese et al., Applied Biochem. Biotech., Vol. 11, 141-152 (1995), PEG-IL-2-Konjugate oder PEG-IFN-β-Konjugate in US 4,766,106, PEG-Polymyxin-Konjugate in WO 90/15628 und PEG-IL-2-Konjugate in WO 90/07939. Einige Konjugate befinden sich inzwischen in der klinischen Anwendung. Beispielsweise konnten die Eigenschaften des Enzyms Asparaginase durch Konjugatbildung mit PEG verbessert werden, und ein PEG-Asparaginase-Konjugat ist unter der Marke Oncaspar® für die Krebstherapie kommerziell erhältlich. Kürzlich wurde ein an PEG gekoppeltes G-CSF von der US Food and Drug Administration zugelassen (Pegfilgastim). Eine große Anzahl weiterer pegylierter Produkte befindet sich in den verschiedenen Phasen der klinischen Entwicklung, darunter beispielsweise PEG-CDP870, PEG-Dronabinol, etc. (vgl. PEG-Pipeline unter www.enzon.com oder www.inhale.com).

Nicht nur Proteine sondern auch andere Verbindungen wurden nach diesem Schema an PEG und andere Polymere gekoppelt. WO 97/33552 und WO 97/38727 offenbaren beispielsweise die Kopplung von Paclitaxel an PEG und die Verwendung des Konjugates zur Behandlung von Tumoren. Die Verwendung eines PEG-Camptothecin-Konjugates zur Behandlung von Tumoren wird von der Firma Enzon in der klinischen Phase I untersucht.

Auch Antibiotika sind bereits an PEG gekoppelt worden. Dowling und Russell berichten beispielsweise über die Pharmakomkinetik eines Oxytetrazyklin-PEG-Konjugates (J. Vet. Pharmacol. Ther., Vol. 23 (2000), 107-110). Für den Erhalt neuer Funktionen wurden Antibiotika im Stand der Technik auch mittels anderer Verfahren derivatisiert. Beispielsweise wurde ein Depot-Penicillin hergestellt, welches ein Procain-Penicillin-Derivat ist, also das Salz des Penicillins mit der Procain-Base. Dieses Derivat besitzt eine verlängerte Wirkungsdauer und wird z.B. zur Therapie der Syphillis eingesetzt.

Kopplungsreaktionen mit mehr als zwei Verbindungen sind ebenfalls beschrieben worden. WO 93/23062 offenbart beispielsweise die Herstellung eines Kopplungsproduktes aus einem gegen ein B-Zell-Lymphom gerichteten Antikörper, aktiviertem PEG und einem Toxin.

PEG-Wirkstoff-Konjugate weisen jedoch keine natürliche Struktur auf, für die in vivo Abbauwege beschrieben wurden. Unter anderem aus diesem Grund sind neben den PEG-Konjugaten auch andere Konjugate und Protein-Polymerisate für die Lösung der oben genannten Probleme erzeugt worden. So gibt es eine Vielzahl von Verfahren zur Vernetzung verschiedener Proteine und Bindung von Proteinen an Makromoleküle (vgl. zusammenfassende Darstellung in Wong, S.S., "Chemistry of protein conjugation and cross linking" , CRCS, Inc. (1993)).

Hydroxyethylstärke (HES) wird als Derivat eines natürlich vorkommenden Amylopektins im Körper durch α-Amylase abgebaut. Auch die Herstellung von HES-Protein-Konjugaten ist bereits im Stand der Technik beschrieben worden (vgl. HES-Hämoglobin-Konjugate in DE 26 16 086 oder DE 26 46 854).

Hämoglobin ist ein Protein, das als Blutersatz- und Sauerstoff-transport-Mittel (sog. "Hämoglobin-Based-Oxygen Carrier", HBOC) eine große klinische Bedeutung haben könnte. Obgleich der Bedarf an einem derartigen Produkt jedoch bereits frühzeitig erkannt wurde (vgl. Rabiner, J. Exp. Med. 126, (1967) 1127), hat bisher keines der bekannten HBOC-Produkte den Status eines zugelassenen Arzneimittels erreicht.

Das natürliche Hämoglobin besteht aus zwei α- und β-Peptidketten, die als prosthetische Gruppe jeweils ein Häm gebunden haben. Isolierte Hämoglobin-Moleküle sind jedoch sehr instabil und zerfallen rasch in die stabileren α,β-Dimere (MG 32 kDa). Die biologische Halbwertszeit von isoliertem Hämoglobin im Blutkreislauf liegt bei etwa 1 Stunde, da die Dimere schnell über die Nieren ausgeschieden werden. Dabei erzeugen die Dimere nephrotoxische Nebenwirkungen (vgl. Bunn & Jandl, J. Exp. Med. 129, (1967) 925-934). Entwicklungsarbeiten an derivatisierten Hämoglobin-Molekülen waren daher in erster Linie darauf gerichtet, Hämoglobin intramolekular zu vernetzen, zur Bildung von polymeren HBOC-Formen intermolekular zu verknüpfen und/oder an Polymere zu koppeln.

Die bekannten Hämoglobin-Konjugate werden beispielsweise in Xue und Wong (Meth. in Enzymol., 231 (1994), S. 308-322) und in DE 26 16 086 und DE 26 46 854 beschrieben. Letztere offenbart Verfahren mittels derer Hämoglobin an HES gebunden wird, indem HES zunächst mit Natriumperiodat umgesetzt wird. Dabei entstehen Dialdehyde, an die Hämoglobin gebunden wird. Demgegenüber beschreibt die DE 26 16 086 die Kopplung von Hämoglobin an HES nach einem Verfahren, bei dem zunächst ein Vernetzungsmittel (z.B. Bromcyan) an HES gebunden wird und anschließend Hämoglobin an das Zwischenprodukt gebunden wird.

HES ist ein substituiertes Derivat des in Maisstärke zu 95 % vorkommenden Kohlenhydrat-Polymers Amylopektin. HES weist vorteilhafte rheologische Eigenschaften auf und wird zurzeit als Volumenersatzmittel und zur Hämodilutionstherapie klinisch eingesetzt (Sommermeyer et al., Krankenhauspharmazie, Vol. 8(8), (1987), S.271-278; und Weidler et al., Arzneim.-Forschung/ Drug Res., 41, (1991) 494-498).

Amylopektin besteht aus Glucoseeinheiten, wobei in den Hauptketten α-1,4-glykosidische Bindungen vorliegen, an den Verzweigungsstellen jedoch α-1,6-glykosidische Bindungen. Die physikalisch-chemischen Eigenschaften dieses Moleküls werden im Wesentlichen durch die Art der glykosidischen Bindungen bestimmt. Aufgrund der abgeknickten α-1,4-glykosidischen Bindung entstehen helikale Strukturen mit etwa 6 Glucose-Monomeren pro Windung.

Die physikalisch-chemischen als auch die biochemischen Eigenschaften des Polymers können durch Substitution verändert werden. Die Einführung einer Hydroxyethylgruppe kann durch alkalische Hydroxyethylierung erreicht werden. Durch die Reaktionsbedingungen kann die unterschiedliche Reaktivität der jeweiligen Hydroxylgruppe im unsubstituierten Glucosemonomer gegenüber der Hydroxyethylierung ausgenutzt werden, dadurch ist eine begrenzte Einflussnahme auf das Substitutionsmuster möglich.

Daher wird HES im Wesentlichen über die Molekulargewichtsverteilung und den Substitutionsgrad gekennzeichnet. Der Substitutionsgrad kann dabei als DS ("degree of substitution"), welcher auf den Anteil der substituierten Glucosemonomere aller Glucoseeinheiten Bezug nimmt, oder als MS ("molar substitution") beschrieben werden, womit die Anzahl von Hydroxyethylgruppen pro Glucoseeinheit bezeichnet wird.

HES-Lösungen liegen als polydisperse Zusammensetzungen vor, in denen sich die einzelnen Moleküle hinsichtlich des Polymerisationsgrades, der Anzahl und Anordnung der Verzweigungsstellen, sowie ihres Substitutionsmusters voneinander unterscheiden. HES ist somit ein Gemisch von Verbindungen mit unterschiedlichem Molekulargewicht. Dementsprechend wird eine bestimmte HES-Lösung durch ein durchschnittliches Molekulargewicht anhand statistischer Größen bestimmt. Dabei wird Mₙ als einfaches arithmetisches Mittel in Abhängigkeit von der Anzahl der Moleküle errechnet (Zahlenmittel), während M_{w}, das Gewichtsmittel, die masseabhängige Messgröße darstellt.

Eine selektive chemische Bindung von Proteinen an HES wurde bisher jedoch dadurch verhindert, dass die Aktivierung der HES nicht selektiv erfolgt. So resultieren die im Stand der Technik bekannten Protein-HES-Konjugate aus einer nicht-selektiven Kopplung von Bromcyan-aktivierter HES an Hämoglobin (vgl. DE 26 16 086). Entsprechende Verfahren können zu polydispersen Produkten mit uneinheitlichen Eigenschaften und potentiell toxischen Nebenwirkungen führen.

Von Hashimoto (Hashimoto et al., Kunststoffe, Kautschuk, Fasern, Vol. 9, (1992) S. 1271-1279) wurde erstmals ein Verfahren offenbart, in dem eine reduzierende Aldehyd-Endgruppe eines Saccharids selektiv oxidiert werden konnte, wobei ein reaktiver Ester (Lacton) gewonnen wurde.

Auf der Grundlage dieses Verfahrens offenbart WO 98/01158, dass Hämoglobin-Hydroxyethylstärke-Konjugate erhalten werden können, in denen Hämoglobin an HES selektiv über Amidbindungen zwischen freien Aminogruppen des Hämoglobins und der in oxidierter Form vorliegenden reduzierenden Endgruppe der HES miteinander verknüpft sind. Sowohl die in Hashimoto et al. offenbarten Verfahren als auch die Verfahren gemäß WO 98/01158 basieren jedoch auf einer Reaktion zwischen Saccharid (HES) und Protein (Hämoglobin) in organischem Lösungsmittel. Konkret wurde in dieser Veröffentlichung Dimethylsulfoxid (DMSO) verwendet.

Dem Fachmann ist jedoch bekannt, dass viele Proteine in organischen Lösungsmitteln eine Strukturänderung erleiden, welche sich in wässrigen Lösungen nicht zurückbildet. In der Regel geht mit der Strukturänderung auch ein Aktivitätsverlust einher. In jedem Fall ist das organische Lösungsmittel aufwendig zu entfernen, da für die geplante medizinische Verwendung bereits residuale Anteile organischer Lösungsmittel nicht akzeptabel sein können. Sogar die potentielle Gefahr von Verunreinigungen und Strukturänderungen der Proteine ist im Hinblick auf die geplante Verwendung auszuschliessen.

Der vorliegenden Erfindung liegt somit die Aufgabe zugrunde, verbesserte Hydroxyalkylstärke-Wirkstoff-Konjugate und Verfahren zur deren Herstellung zur Verfügung zu stellen, die zu biologisch aktiven Konjugaten führen, welche im klinischen Alltag eingesetzt werden können. Eine weitere Aufgabe der vorliegenden Erfindung besteht darin, ein Verfahren zur Herstellung von Hydroxyalkylstärke-Wirkstoff-Konjugaten bereit zu stellen, bei denen nicht in nennenswertem Umfang Nebenprodukte erzeugt werden, da auch diese Nebenprodukte die anschließende Aufreinigung des Produktes in erheblichem Umfang beeinträchtigen.

Diese Aufgabe wurde nunmehr überraschenderweise durch Verbindungen gelöst, die ein Konjugat aus einer Hydroxyalkylstärke und einem Wirkstoff umfassen, wobei die Hydroxyalkylstärke entweder unmittelbar oder über einen Linker kovalent an den Wirkstoff gebunden ist. Entsprechende HAS-Wirkstoff-Konjugate sind beispielsweise durch Verfahren erhältlich, bei denen man HAS und einen Wirkstoff in einem Reaktionsmedium miteinander umsetzt, wobei das Reaktionsmedium Wasser oder ein Gemisch von Wasser mit organischem Lösungsmittel, welches mindestens 10 Gew.-% Wasser umfaßt, ist.

Die Erfindung betrifft ferner Verfahren zur Herstellung eines kovalenten HAS-Wirkstoff-Konjugates, bei dem man HAS und mindestens einen Wirkstoff in einem wässrigen Reaktionsmedium miteinander umsetzt und ist dadurch gekennzeichnet, dass das Reaktionsmedium Wasser oder ein Gemisch von Wasser mit organischem Lösungsmittel, welches mindestens 10 Gew.-% Wasser umfaßt, ist.

Vorzugsweise wird HAS vor Bindung an den Wirkstoff oxidiert, wobei eine spezifische Oxidation der reduzierenden Endgruppen besonders bevorzugt ist. Alternativ dazu kann die Kopplung über die Bildung einer Schiff'schen Base zwischen HAS und einem AminGruppen-tragenden Wirkstoff als Zwischenprodukt erfolgen. Dieses Zwischenprodukt wird anschließend unter Ausbildung einer Methylenamingruppe reduziert.

### KURZE BESCHREIBUNG DER FIGUREN:

- Fig. 1: GPC Chromatogramm der Kopplungsreaktion zwischen ox-HES 130 kD und HSA nach Verfahren A.III;
- Fig. 2: GPC Chromatogramm der Kopplungsreaktion zwischen ox-HES 130 kD und HSA nach Verfahren A.IV;
- Fig. 3: GPC Chromatogramm der Kopplungsreaktion zwischen ox-HES 130 kD und HSA nach Verfahren A.V und einer Reaktionszeit von 2 Stunden;
- Fig. 4: GPC Chromatogramm der Kopplungsreaktion zwischen ox-HES 130 kD und HSA, Verfahren A.V, Reaktionszeit über Nacht;
- Fig.5: GPC Chromatogramm der Kopplungsreaktion zwischen ox-HES 10 kD und HSA nach Verfahren A.V nach 2 Stunden (Fig. 5a) und über Nacht (Fig. 5b);
- Fig. 6: GPC Chromatogramm der Kopplungsreaktion zwischen ox-HES 130 kD und HSA nach Verfahren A.VII, nach 24 Stunden Reaktionszeit;
- Fig. 7: GPC Chromatogramm der Kopplungsreaktion zwischen ox-HES 130 kD und HSA nach Verfahren B.V;
- Fig. 8: SDS-PAGE und Western Blot verschiedener Kopplungsreaktionen zwischen HES und HSA;
- Fig. 9: SDS-PAGE und Western Blot verschiedener Kopplungsreaktionen zwischen HES und HSA;
- Fig.10: Reaktionsschema für die Erstellung eines HES-DNA-Konjugates;
- Fig.11: Aufnahme eines Gels, das HES-DNA-Konjugate vor und nach einem Verdau mit einem Restriktionsenzym zeigt.

Die vorliegende Erfindung stellt erstmals Verbindungen zur Verfügung, die ein Konjugat aus Hydroxyalkylstärke und einem Wirkstoff umfassen, wobei die Hydroxyalkylstärke entweder unmittelbar oder über einen Linker kovalent an den wirkstoff gebunden ist. Die vorliegende Erfindung stellt ferner HAS-Wirkstoff-Konjugate zur Verfügung, welche durch Verfahren herstellbar sind, bei denen man HAS und mindestens einen Wirkstoff in einem wässrigen Reaktionsmedium miteinander umsetzt. Die Verfahren sind ferner dadurch gekennzeichnet, dass das Reaktionsmedium Wasser oder ein Gemisch von Wasser mit organischem Lösungsmittel, welches mindestens 10 Gew.-% Wasser umfaßt, ist.

Im Rahmen der vorliegenden Erfindung wird eine chemische Verbindung als Wirkstoff bezeichnet, wenn die Verbindung geeignet ist, aktiver Bestandteil eines beliebigen Mittels für therapeutische oder diagnostische Zwecke zu sein. Vorzugsweise handelt es sich bei dem Wirkstoff um ein den aktiven Bestandteil eines Arzneimittels, also die Verbindung innerhalb einer Arzneimittelformulierung, welche nach Verabreichung an ein Subjekt eine physiolgische Wirkung erzielt.

Eine Übersicht über die zugelassenen Arzneimittel und deren Wirkstoffe findet sich in der Roten Liste. Die in der Roten Liste genannten Wirkstoffe können für die Herstellung der HAS-Wirkstoff-Konjugate nach dem erfindungsgemäßen Verfahren verwendet werden. Gemäß der vorliegenden Erfindung umfaßt der Begriff Wirkstoff aber auch alle Verbindungen, deren Eignung für die diagnostische oder therapeutische Verwendung zwar bekannt ist, die jedoch aufgrund der oben dargestellten Probleme bisher für diesen Zweck nicht eingesetzt werden konnten. Vorzugsweise handelt es sich bei dem Wirkstoff um ein Vitamin, Impfstoff, Toxin, Antibiotikum (oder Antiinfektivum), Antiarrhytmikum, Appetitzügler, Anästhetikum, Analgetikum, Antirheumatikum, Antiallergikum, Antiasthmatikum, Antidepressivum, Antidiabetikum, Antihistaminikum, Antihypertonikum, oder ein antineoplastisches Mittel. Strukturell kann es sich beispielsweise um ein Hormon, Steroid, Lipid, Protein, Oligo- oder Polypeptid, eine Nukleinsäure, insbesondere eine D- oder L-Nukleinsäure, wie beispielsweise eine D-DNA, L-DNA, D-RNA oder L-RNA handeln. Die Verwendung von Proteinen, Peptiden, D- oder L-Nukleinsäuren als HAS-Kopplungspartner ist besonders bevorzugt.

Die nach der vorliegenden Erfindung hergestellten Verbindungen behalten die Aktivität des Wirkstoffes und die vorteilhaften Eigenschaften der HAS. Als weitere Vorteile weisen die erfindungsgemäßen Konjugate weitere Vorteile, darunter eine verbesserte in vivo Halbwertszeit der Wirkstoffe, eine verringerte Toxizität, eine verbesserte Stabilität und/oder eine verbesserte Löslichkeit der Wirkstoffe auf.

Die HAS-Kette wird im Plasma nach der Verabreichung durch alpha-Amylase verkürzt. Die Aktivität des Kopplungsproduktes kann somit als Aktivität des nativen Kopplungsproduktes, also unmittelbar nach der Kopplung, oder als Aktivität des metabolisierten Kopplungsproduktes, also nach in vivo Metabolisierung des Kopplungsproduktes, bestimmt werden. Die in vivo Metabolisierung kann durch einen in vitro Abbau simuliert werden.

Die Aktivität des Wirkstoffes kann nach im Stand der Technik für diesen Stoff bekannten Verfahren bestimmt werden. Bei einem antineoplastischen Mittel wird die Aktivität beispielsweise als inhibitorische Konzentration (IC) oder bei einem antiinfektiven Mittel als minimale Hemmkonzentration (MHK) bestimmt. Die Bestimmung erfolgt vorzugsweise in vitro an geeigneten Zielzellen (vgl. Chow et al., Haematologica, Vol. 86 (2001), S. 485-493). Die in vitro Effekte können ferner durch ein relevantes Tiermodell bestätigt werden (vgl. beispielsweise das Mausmodell des Nierenzellkarzinoms, das in Changnon et al. beschrieben wurde, vgl. BJU Int., Vol. 88 (2001), S. 418-424).

Im Vergleich zu der ungekoppelten Substanz kann das native Kopplungsprodukt eine gesteigerte oder reduzierte Aktivität aufweisen. Die Aktivität wird jedoch vorzugsweise nicht mehr als 5fach, besonders bevorzugt nicht mehr als 3- oder 2-fach reduziert. Das metabolisierte Produkt weist vorzugsweise eine mit der ungekoppelten Substanz vergleichbare Aktivität auf, d.h. das metabolisierte Konjugat weist mindestens 50%, vorzugsweise mindestens 75% der Aktivität des Wirkstoffes vor der Kopplung auf, wobei ein Erhalt von mindestens 95% der Aktivität besonders bevorzugt ist.

Im Rahmen der vorliegenden Erfindung wird der Begriff "Hydroxyalkylstärke" dazu verwendet, um Stärkederivate zu bezeichnen, die mit einer Hydroxyalkylgruppe mit 1 bis 3 C-Atomen substituiert wurden. Die als "Hydroxyalkylstärke" bezeichnete Gruppe umfaßt somit Hydroxymethylstärke, Hydroxyethylstärke und Hydroxypropylstärke. Die Verwendung von Hydroxyethylstärke (HES) als Kopplungspartner ist für alle Ausführungsformen der Erfindung besonders bevorzugt.

Erfindungsgemäß ist es bevorzugt, dass die Hydroxyethylstärke ein mittleres Molekulargewicht (Gewichtsmittel) von 1-300 kDa, wobei ein mittleres Molekulargewicht von 5 bis 200 kDa besonders bevorzugt ist. Hydroxyethylstärke kann ferner einen molaren Substitutionsgrad von 0,1-0,8 und ein Verhältnis von C₂:C₆₋Substitution im Bereich von 2-20, jeweils bezogen auf die Hydroxyethylgruppen, aufweisen.

Für die Kopplung des Wirkstoffes an die HAS kann es in einem ersten Schritt notwendig sein, eine aktive Gruppe in den Wirkstoff und/oder in die HAS einzuführen. Entsprechende aktive Gruppen können beispielsweise Thiol- oder Aminogruppen sein (vgl. Beispiele).

Ferner können Wirkstoff und HAS unter Verwendung eines Linkers aneinander gekoppelt werden. Als Linker kann ein beliebiges Vernetzungsmittel eingesetzt werden. Zahlreiche Vernetzungsmittel, wie beispielsweise SMCC (Succinimidyl-4-(N-maleimidomethyl)cyclohexan-1-carboxylat; vgl. Beispiel 7), sind kommeriziell erhältlich und dem Fachmann geläufig (vgl. alphabetische Liste der "Cross-linking Reagents" im Produktkatalog der Firma Perbio und www.piercenet.com).

Gemäß einer Ausführungsform der vorliegenden Erfindung sind wasserlösliche, einen Aminozucker aufweisende Antibiotika-Derivate, insbesondere HAS-Daunorubicin und HAS-Doxorubicin-Konjugate, sowie Verfahren zu deren Herstellung, soweit in DE 101 29 369 offenbart, nicht von der vorliegenden Erfindung umfaßt.

Gemäß einer bevorzugten Ausführungsform betrifft die vorliegende Erfindung Verbindungen, die ein Konjugat aus HAS und einem antineoplastischen Wirkstoff umfassen und deren Verwendung zur Behandlung von Tumoren.

Tumorzellen unterscheiden sich von normalen Körperzellen u.a. dadurch, daß die Tumorzellen einer physiologischen Wachstumskontrolle entzogen sind und daher eine gesteigerte Zellteilurigsrate aufweisen. Die therapeutische Verwendung antineoplastischer Wirkstoffe bei der Tumortherapie basiert auf diesem Unterschied, da sich die toxische Aktivität antineoplastischer Wirkstoffe primär gegen proliferierende Zellen richtet. Verbindungen werden daher als antineoplastische Wirkstoffe oder Zytostatika bezeichnet, wenn sie eine toxische Aktivität gegen proliferierende Zellen aufweisen (Grundlagen der Onkologie und gegenwärtige Therapieansätze werden beispielsweise in Internistische Onkologie, Schmoll et al. (Herausg.), Springer, 1996, zusammengefaßt).

Chemisch können die antineoplastischen Wirkstoffe einer sehr heterogenen Gruppe zugeordnet werden. Neben der Proliferationshemmung gewinnt in der Diskussion der letzten Jahre die Apoptoseinduktion, der programmierte Zelltod, an Bedeutung. Eine Klassifikation der antineoplastischen Wirkstoffe kann beispielsweise über die kritischen Zielmoleküle erfolgen (Schmoll et al., a.a.O.):
(1) Verbindungen, welche die DNS-Biosynthese hemmen, beispielsweise durch Antimetaboliten, wie MTX, 5-FU, Ara-C oder Hydroxyharnstoff.
(2) Verbindungen, welche auf die DNS einwirken, beispielsweise durch Induktion von Strangbrüchen, Interkalation, Veränderung der Zwischenstrangvernetzung, Topoisomerasegifte, wie Alkylantien, Platinkomplexe Anthrazykline, Bleomycin, Actinomycin D oder Epipodophyllotoxine.
(3) Verbindungen, welche auf die RNS einwirken, beispielsweise durch Blockade der mRNS Synthese durch Interkalation oder Einbau in die RNS, darunter Anthrazykline, Bleomycin, Actinomycin D oder Antimetabolite.
(4) Verbindungen, welche auf Proteine einwirken, beispielsweise auf der Ebene der Rezeptorbindung (z.B. Hormone oder Antagonisten), durch Inhibition der Tubulinpolymerisation (z.B. mittles Vincaalkaloiden), durch Proteinvernetzung (z.B. mittels Alkylantien) oder Phosphorylierung (z.B. mittels Proteinkinase-C-Inhibitoren).

Aufgrund der antineoplastischen Aktivität weisen alle Wirkstoffe erhebliche Nebenwirkungen auf, die sich primär als Hemmung rasch proliferierender Gewebe bemerkbar machen. So ist insbesondere die Erythro-, Leuko- und Thrombopoese gehemmt, sowie das Wachstum der Schleimhautepithelien beeinträchtigt. Als Folge können gastrointestinale Störungen oder irreversible Beeinträchtigungen der Spermatogenese bzw. Anovolation eintreten. Auch die Haut und Hautanhangsgebilde sind regelmäßig betroffen, beispielsweise erleiden viele Patienten einen reversiblen Haarausfall.

In schweren Fällen können die Nebenwirkungen bis hin zu akutem Nierenversagen und toxisch bedingten Organschädigungen an Herz, Lunge, Leber und Nervensystem führen. Schließlich muß als Folge der immunsuppressiven Wirkung mit einer gehäuften Anzahl von Infektionen gerechnet werden.

Die Herstellung und Erforschung von Konjugaten, die einen antineoplastischen Wirkstoff enthalten, war daher auf das Ziel gerichtet, die Verträglichkeit des Wirkstoffes zu verbessern. Für diesen Zweck sind verschiedene antineoplastische Wirkstoffe an Makromoleküle, beispielsweise an Dextran gekoppelt worden (vgl. Kojima et al., J.Pharm.Pharmakol., Vol. 32 (1980), S.30-34; Nakane et al., J.Pharm.Pharmakol., Vol.40 (1988), S.1-6; Nomura et al., J.Controlled Release, Vol.52 (1998), S.239-252; Sato et al., J.Pharm.Sci., Vol.78 (1989), S.11-16). Eine verbesserte Antitumorwirkung der Konjugate konnte in einigen Fällen festgestellt werden.

Alternativ dazu wurden Wirkstoffe, wie Mitomycin C, auch an N-Succinylchitosan (Song et al., J.Controlled Release, Vol.42 (1996), S.93-100), Carboxymethylchitin (Song et al., Arch.Pract.Pharm. Vol.53 (1993), S.141-147) und an Oligopeptide gekoppelt (Soyez et al., J.Controlled Release, Vol.47 (1997), S.71-80). In der Mehrzahl der Analysen wurde wiederum eine verbesserte Antitumoraktivität der Konjugate im Vergleich zu dem einzelnen antineoplastischen Wirkstoff beobachtet.

Erfindungsgemäß wurde nunmehr überraschenderweise festgestellt, dass HAS-Wirkstoff-Konjugate, welche einen antineoplastischen Wirkstoff umfassen, eine verbesserte toxische Wirkung gegenüber Tumorzellen und/oder eine verringerte Toxizität für andere Zellen aufweisen. Die Konjugate ermöglichen damit eine größere therapeutische Breite.

Die Plasma-Halbwertszeit der Konjugate wird signifikant verlängert. Dies ermöglicht ein Durchbrechen der Reparaturmechanismen in Tumor-Zellen durch längere Exposition. Gleichzeitig ermöglicht die vorliegende Erfindung ein langsameres Anfluten, insbesondere in gesundem Gewebe, wodurch eine geringere Peak-Konzentration und eine verbesserte Verträglichkeit für den Patienten erreicht wird.

Für die Herstellung der erfindungsgemäßen Konjugate kann ein beliebiger antineoplastischer Wirkstoff verwendet werden. Die antineoplastischen Wirkstoffe können beispielsweise aus den Gruppen bestehend aus Alkylantien, Antimetaboliten, Antibiotika oder Naturstoffen ausgewählt werden.

Gemäß einer bevorzugten Ausführungsform handelt es sich bei dem antineoplastischen Wirkstoff um Mitomycin C, Cyclophosphamid, Bleomycin, Chlorambucil, Cisplatin, Ara-C, Fludarabin, Doxorubicin, Etoposid, 5-FU, MTX, Vinblastin, Vincristin, Vindesin, Hydroxyurea, 6-MP oder CCNU.

Die Verwendung von Mitomycin C als Wirkstoff ist besonders bevorzugt. Mitomycin C gehört zur Gruppe der Antibiotika und enthält eine Aziridin- sowie eine Chinon-Gruppe und einen Mitosan-Ring. Der Wirkstoff wird zur Behandlung von Nierenzellkarzinomen, bei Blasentumoren und bei anderen urologischen Erkrankungen eingesetzt. Die Verbindung erlangt ihre Aktivität erst nach Metabolisierung in hypoxischen (also bevorzugt Tumor-) Zellen durch intrazelluläre enzymatische oder spontane chemische Reduktion des Chinons und Verlust der Methoxy-Gruppe. An diese Methoxy-Gruppe kann HAS bevorzugt über einen Linker gekoppelt werden. Nach Abspaltung des Substituenten in der Zelle liegt intrazellulär der gleiche Wirkstoff vor, der eine alkylierende Vernetzung der DNA bewirkt und dadurch eine toxische Wirkung entfaltet. Alternativ dazu könnte HAS auch an eine der beiden NH₂-Gruppen gekoppelt werden. Mitomycin C weist eine typische Gewebe-Spezifität auf. Erfindungsgemäß ist es bevorzugt, dass diese Spezifität - insbesondere für exkretorische Organe - durch HAS-Kopplung erhöht wird.

Der antineoplastische Wirkstoff kann erfindungsgemäß nach beliebigen Verfahren an HAS gekoppelt werden. Eine spezifische Kopplung an die reduzierenden Endgruppen der HAS ist jedoch bevorzugt, da durch dieses Vorgehen ein definiertes Konjugat erzeugt wird.

Gemäß einer Ausführungsform der Erfindung wird Hydroxyethylstärke an die Methoxy-Gruppe des Mitomycin C gekoppelt. Dabei kann die Kopplung an die Methoxy-Gruppe des Mitomycin C über einen Linker erfolgen.

Gemäß einer weiteren Ausführungsform betrifft die vorliegende Erfindung Verfahren zur Herstellung einer Verbindung, die ein Konjugat aus HAS und einem antineoplastischen Wirkstoff umfaßt. Das Verfahren umfaßt Schritte, bei denen man HAS entweder unmittelbar oder über einen Linker kovalent an einen antineoplastischen Wirkstoff koppelt und das Konjugat isoliert.

Ferner betrifft die Erfindung pharmazeutische Zusammensetzungen, welche eine Verbindung umfassen, die ein Konjugat aus HAS und einem antineoplastischen Wirkstoff aufweisen. Die pharmazeutische Zusammensetzung kann ferner einen pharmazeutisch verträglichen Träger und/oder ein Zytokin umfassen. Das Zytokin ist vorzugsweise IL-2, α-Interferon, γ-Interferon.

Die pharmazeutische Zusammensetzung kann in einer beliebigen im Stand der Technik bekannten Applikationsform vorliegen. Beispielsweise kann die Zusammensetzung unter Umständen zur oralen oder parenteralen Verabreichung formuliert sein. Die Formulierung der Zusammensetzung erfolgt nach im Stand der Technik üblichen Verfahren. Neben dem Wirkstoff enthält die Zusammensetzung üblicherweise einen pharmazeutisch verträglichen Träger und einen oder mehrere Hilfsstoffe, sowie gegebenenfalls Konservierungsmittel, Lösungsvermittler, etc.

Schließlich betrifft die vorliegende Erfindung die Verwendung einer Verbindung, die ein Konjugat aus HAS und einem antineoplastischen Wirkstoff umfaßt, zur Herstellung eines Arzneimittels zur Behandlung von Tumoren und/oder deren Metastasen, insbesondere zur Behandlung von urologischen Tumoren und/oder Metastasen urologischer Tumore, zur Behandlung von Metastasen des Nierenzellkarzinoms, oder zur Behandlung lymphatischer Systemerkrankungen, wie beispielsweise CLL, Hodgkin-Lymphom, NHL, multiples Myelom, Morbus Waldenström. Auch gemäß dieser Ausführungsform der Erfindung kann das Arzneimittel ferner ein Zytokin, beispielsweise IL-2, α-Interferon, γ-Interferon, umfassen.

Die Verwendung der erfindungsgemäßen Verbindungen zur Herstellung eines Arzneimittels zur Behandlung von urologischen Tumoren und/oder Metastasen urologischer·Tumore, beispielsweise zur Behandlung von Metastasen des Nierenzellkarzinoms ist besonders bevorzugt. Eine kurative Therapie des Nierenzellkarzinoms ist zur Zeit weder mit einer Kombinations-Chemotherapie noch mit Mitomycin C alleine zu erreichen. Dies kann an der ungünstigen Pharmakokinetik der Verbindung liegen, da der Anteil der renalen Elimination nur bei ca. 18% liegt. Da HAS nahezu vollständig über die Nieren eliminiert wird, weist das Konjugat einen höheren Prozentsatz der renalen Elimination im Vergleich zu der nichtkonjugierten Substanz auf. Diese Ausführungsform der vorliegenden Erfindung nutzt die intrazelluläre Zwischenspeicherung der HAS. Insbesondere hoch-substituierte HAS-Sorten (HAS 200/0,62) weisen eine verstärkte intrazelluläre Speicherung, im Extremfall sogar eine Überladung auf. Dieses Phänomen ist auch im Bereich des proximalen Tubulus beobachtet worden (Peron et al., Clinical Nephrology, Vol. 55 (2001), S.408-411).

Gemäß dieser Ausführungsform stellt die vorliegende Erfindung somit eine Anreicherung eines antineoplastischen Wirkstoffs in bestimmten Zielzellen oder -geweben zur Verfügung. Die verbesserte Pharmakokinetik der Konjugate ermöglicht es somit bei niedrigeren systemischen Konzentrationen eine wesentlich höhere Konzentration in den Zellen des Zielorgans zu erreichen. Diese medizinische Verwendung wird bevorzugt bei dem klarzelligen und dem chromophilen Nierenkarzinom eingesetzt, welche zusammen etwa 90% aller histologischer Typen ausmachen.

Gemäß einer alternativen Ausführungsform betrifft die Erfindung die Verwendung der erfindungsgemäßen Verbindungen zur Herstellung eines Arzneimittels zur Behandlung lymphatischer Systemerkrankungen, beispielsweise CLL, Hodgkin-Lymphom, NHL, multiples Myelom, Morbus Waldenström. Durch die erfindungsgemäße Kopplung von HAS an einen antineoplastischen Wirkstoff wird die intrazelluläre Aufnahme der Wirkstoffe in Abhängigkeit der Kettenlänge und des Sustitutionsgrads verlangsamt. Ferner haben radioaktive kinetische Untersuchungen gezeigt, dass HAS in bestimmten Organen, darunter lymphatische Organe, länger als im Gesamtkörper gespeichert wird (vgl. Bepperling et al., Crit. Care, Vol.3, Suppl. 1 (1999), S.153). Es erfolgt somit eine Anreicherung der Konjugate in den Zielzellen, was eine verbesserte Pharmakokinetik bei geringerer systemischer Toxizität zur Folge hat.

Die Behandlung lymphatischer Systemerkrankungen unter Verwendung von Fludarabin als Wirkstoff ist bevorzugt. Fludarabin ist ein halogeniertes Adeninanalog, das gegenüber einer Deaminierung resistent ist.

Die Erfindung betrifft ferner die Verwendung der erfindungsgemäßen Verbindungen zur Herstellung eines Arzneimittels zur Behandlung von kutanen/lokalen primären Malignomen oder deren Metastasen. Dabei werden zwei Effekte genutzt, die gezielte, gesteigerte Aufnahme durch die genannten Gewebe und der verzögerte Abtransport des HAS-Konjugates aus dem Gewebe. Beides führt zu einer Anreicherung des Konjugates in den Zielzellen.

Die Erfindung betrifft ferner die Verwendung der erfindungsgemäßen Verbindungen zur Herstellung eines Arzneimittels zur Behandlung von hämatologischen Systemerkrankungen oder onkologischen Erkrankungen, beispielsweise von nicht-kleinzelligen und kleinzelligen Bronchialkarzinomen, Mammakarzinomen, ösophagus-Plattenepithelkarzinomen, Nierenzellkarzinomen, Hodenkarzinomen, maligne Melanome, ALL oder CML. Insbesondere bei der Verwendung der Konjugate zur Behandlung des Nierenzellkarzinoms ergeben sich Vorteile aufgrund der starken Anreicherung der Verbindung in dem betroffenen Gewebe durch die größere Hydrophilie des Konjugates und der daraus folgenden stärkeren renalen Elimination. Für diese Ausführungsform der Erfindung ist die Verwendung von Vindesin als Wirkstoff besonders bevorzugt.

Die Erfindung betrifft ferner die Verwendung der erfindungsgemäßen Verbindungen zur Herstellung eines Arzneimittels, wobei die Verbindung als Kombinationstherapie mit einem oder mehreren weiteren antineoplastischen Wirkstoffen oder Zytokinen eingesetzt wird. Die Kombinationstherapie kann durch Verabreichung eines Mittels erfolgen, in dem alle Wirkstoffe vorliegen, oder durch Verabreichung von zwei verschiedenen oder mehr Mitteln, in denen jeweils ein Wirkstoff formuliert wurde.

Die vorliegende Erfindung stellt ferner Verfahren zur Herstellung eines Arzneimittels zur Verfügung, das ein Zytokin und eine erfindungsgemäße Verbindung enthält und für neue Kombinationstherapien geeignet ist. Entsprechende Mittel sind insbesondere für die Behandlung des fortgeschrittenen Nierenzellkarzinoms geeignet.

Gemäß einer weiteren besonders bevorzugten Ausführungsform der Erfindung werden Konjugate aus HAS und einem antiarrhythmischen Wirkstoff bereitgestellt, sowie deren Verwendung zur Behandlung von Herzrhythmusstörungen.

Abweichungen in der zeitlichen Folge und Regelmäßigkeit der Herzaktionen (Arrhythmien) von der normalen Herzfrequenz werden als Herzrhythmusstörungen bezeichnet. Die Abweichungen sind in der Mehrzahl der Fälle durch Erregungsbildungs- oder Erregungsleitungsstörungen des Herzens bedingt. Als antiarrhythmische Wirkstoffe oder Antiarrhythmika werden Substanzen bezeichnet, die zur Behandlung von Herzrhythmusstörungen, insbesondere bei ventrikulären Herzrhythmusstörungen geeignet sind.

Je nach Art der Wirkung der antiarrhythmischen Wirkstoffe unterscheidet man zwischen Natriumkanal-Blockern (Chinidin, Procainamid, Disopyramid, etc.), Beta-Rezeptoren-Blockern (Atenolol, Propanolol, etc.), selektiven Repolarisationsverlängerern (Amiodaron, Sutalol, etc.), Calcium-Antagonisten (Verapamil, Gallopamil, etc.) und Lokalanästhetika.

Die im Stand der Technik üblichen antiarrhythmischen Wirkstoffe, weisen jedoch teilweise eine kurze Wirkungszeit auf. Adenosin beispielsweise ist ein antiarrhythmischer Wirkstoff mit sehr kurzer Halbwertszeit. Die Wirkdauer dieses Stoffes beträgt nur wenige Minuten. Eine Verlängerung der Halbwertszeit und der Wirkdauer ist in vielen Fällen notwendig.

Einige antiarrhythmische Wirkstoffe weisen zudem pro-arrhytmogene Nebenwirkungen, teilweise sogar eine erhöhte Mortalität, auf.

Die vorliegende Erfindung stellt unter anderem verbesserte antiarrhythmische Wirkstoffe zur Verfügung, die beispielsweise verlängerte Wirkdauer aufweisen. Erfindungsgemäß wurde überraschenderweise festgestellt, dass die HAS-Antiarrhythmika-Konjugate eine signifikant längere in vivo Plasma-Halbwertszeit aufweisen und die Aktivität der Wirkstoffe durch die Kopplung an HAS nicht wesentlich beeinträchtigt wird.

Im Rahmen der vorliegenden Erfindung können beliebige antiarrhythmische Wirkstoffe für die Herstellung der Konjugate verwendet werden. Der Wirkstoff kann aus der Gruppe bestehend aus Natriumkanal-Blocker, Beta-Rezeptoren-Blocker, selektive Repolarisationsverlängerer, Calcium-Antagonisten und Lokalanästhetika ausgewählt werden. Vorzugsweise handelt es sich bei dem Wirkstoff um Adenosin, Chinidin, Procainamid, Disopyramid, Lidocain, Phenytoin, Mexiletin, Ajamalin, Parjmalium, Propafenon, Atenolol, Propanolol, Amiodaron, Sotalol, Verapamil, Gallopamil oder Diltiazem, wobei die Verwendung von Adenosin besonders bevorzugt ist.

Gemäß einer Ausführungsform der vorliegenden Erfindung erfolgt die Bindung zwischen dem antiarrythmischen Wirkstoff und der HAS über die reduzierenden Endgruppen der HAS.

Bei Verwendung von Adenosin kann dieser Wirkstoff beispielsweise über die Aminogruppe an die HAS gebunden werden, wobei eine Kopplung zwischen der Aminogruppe des Adenosin und der reduzierenden Endgruppe der HAS besonders bevorzugt ist. Bevorzugt ist eine Kopplungsvariante, bei der nach Metabolisierung (Absprung des HAS) natives Adenosin vorliegt.

Alternativ dazu kann der Wirkstoff über einen sogenannten Linker an die HAS gekoppelt werden.

Die vorliegende Erfindung betrifft ferner pharmazeutische Zusammensetzungen, welche eine der erfindungsgemäßen Verbindungen umfaßt. Die pharmazeutische Zusammensetzung umfaßt ferner üblicherweise einen pharmazeutisch verträglichen Träger und kann beispielsweise für eine intravenöse Applikation formuliert sein.

Schließlich betrifft die Erfindung die Verwendung der erfindungsgemäßen Verbindungen zur Herstellung eines Arzneimittels zur Behandlung von Herzrhythmusstörungen, insbesondere zur Behandlung ventrikulärer Herzrhythmusstörungen.

Gemäß einer alternativen Ausführungsform betrifft die Erfindung die Verwendung einer erfindungsgemäßen Verbindung zur Herstellung eines Arzneimittels zur Induktion von Apoptose, beispielsweise in Tumorgeweben oder in inflammatorischen Geweben.

Die vorliegende Erfindung betrifft Verbindungen, die ein Konjugat aus HAS und einem antiinfektivem Wirkstoff, beziehungsweise einem Antibiotikum umfassen, sowie deren Verwendung zur Behandlung von Infektionserkrankungen.

Das Eindringen von Mikroorganismen (Viren, Bakterien, Pilze, Protozoen) in einen Makroorganismus (Pflanze, Tier, Mensch) und die Vermehrung in diesem wird als Infektion bezeichnet. Entstehung und Verlauf einer Infektionskrankheit hängen im wesentlichen von der Pathogenität des Mikroorganismus und von der Immunität des Makroorganismus ab.

Zur Bekämpfung von Infektionskrankheiten werden seit Jahrzehenten antiinfektive Wirkstoffe als Chemotherapeutika eingesetzt.

Bereits 1928 wurde Penicillin anhand der Eigenschaft des Wirkstoffs, Staphylokokken-freie Zonen auf Kulturschalen zu bilden, von A. Flemings identifiziert. Penicillin war auch das erste Antibiotikum, das im industriellen Maßstab gewonnen werden konnte und große Bedeutung in der klinischen Praxis erlangte.

Als Penicilline werden heute Wirkstoffe aus einer Gruppe von β-Laktam-Antibiotika bezeichnet, die von einem Pilz der Art Penicilium (z.B. P. chrysogenum und P. notatum) gebildet werden. Die bakteriozide Wirkung beruht auf der Blockierung der Synthese der Bakterienzellwand. Das Penicillin inaktiviert das bakterielle Enzym Transpeptidase, wodurch die Quervernetzung der Polysaccharidketten des Mureins der Zellwand verhindert wird.

Seit der Entdeckung wurden zahllose Wirkstoffe isoliert und synthetisiert, die das Wachstum von Mikroorganismen hemmen oder diese abtöten. Die meisten Antibiotika stammen von Streptomyces-Arten (ca. 65%), die aus dem Boden isoliert wurden. Es wird angenommen, daß die Stoffe vom Mikroorganismus zur Unterdrückung von Konkurrenten im Boden benutzt werden.

Die Zahl der isolierten Antibiotika wird auf ca. 8000 geschätzt, von denen ca. 100 in der Medizin anwendbar sind. Eine Einteilung der Wirkstoffe in verschiedene Stoffklassen erfolgte nach unterschiedlichen Gesichtspunkten, beispielsweise nach chemischer Struktur oder Wirkungsmechanismus.

Inzwischen sind Antibiotika nicht nur für die Bekämpfung von Infektionskrankheiten, sondern auch als Immunodepressiva, Zytostatika in der Antitumortherapie, Pflanzenschutzmittel, zur Nahrungsmittel-Konservierung, als Masthilfsmittel in der Tierfütterung, etc. zugelassen.

In den letzten Jahren traten zahlreiche gegen Antibiotika resistente Stämme von Mikroorganismen auf. Dabei wurden neben einfach-resistenten auch häufiger mehrfach-resistente Stämme gefunden, welche die Bekämpfung bestimmter Krankheiten besonders erschweren.

Bei der Untersuchung der Aktivität verschiedener Antibiotika gegen bestimmte Erreger wurde festgestellt, daß einige der Wirkstoffe (beispielsweise Amoxycillin oder Ampicillin) fast ausschließlich extrazellulär wirken (Scaglione et al., Chemotherapie, Vol.39 (1993), 416-423; Balland et al., J.Antimicrob. Chemother. , Vol.37 (1996), 105-115). Diese Wirkstoffe lassen sich somit gegen Mirkoorganismen, die primär intrazellulär vorliegen, nicht einsetzen. Zur Verbesserung der intrazellulären Aktivität wurden Ampicillin-Nanopartikel hergestellt (vgl. Balland et al., a.a.O.).

Bei Infektionen wie Tuberkulose oder anderen Mykobakteriosen wäre eine Erweiterung des Spektrums der Behandlungsmöglichkeiten wegen der immer erforderlichen Kombinationstherapie wünschenswert. Bei anderen interzellulären Infektionen, wie der Chlamydien-Infektion, die in ihrer möglichen Bedeutung für die Pathogenese der Arteriosklerose erst kürzlich erkannt wurde (Stille und Dittmann, Herz, Vol.23 (1998), S.185-192) könnten intrazelluläre, mit Depotwirking versehene Antibiotika einen wesentlichen Fortschritt in Therapie und Prophylaxe bedeuten.

Erfindungsgemäß wurde nunmehr überraschenderweise festgestellt, dass die Kopplung antiinfektiver Wirkstoffe an HAS zu einer Verbesserung der pharmakokinetischen Eigenschaften der Wirkstoffe, insbesondere zu einer verlängerten in vivo Halbwertszeit, einer verbesserten intrazellulären Aufnahme und/oder Wirksamkeit des Wirkstoffs führt.

Erfindungsgemäß kann ein beliebiger antiinfektiver Wirkstoff, bzw. ein beliebiges Antibiotikum verwendet werden. Vorzugsweise wird ein Wirkstoff aus der Gruppe bestehend aus Aminopenicilline, Cephalosporine und Aminocephalosporine, Beta-Laktam-Antibiotika, Carbapeneme, Aminoglycoside, Tetracycline, Makrolid-Antibiotika, Gyrasehemmer, Glycopeptid-Antibiotika, Lincomycine, Streptogramine, Everninomicine, Oxazolidinone, Nitroimidazole, Sulfonamide, Co-trimoxazol, Lokalantibiotika, Virustatika, Antimycotika, Tuberculostatika ausgewählt.

Dabei kann es sich beispielsweise um Ampicillin, Amoxicillin, Cefotaxim, Ceftazidim, Vancomycin, Clindamycin, Metronidazol, Isoniazid, Rifympicin, Rifabutin, Rifapentin, Ethambutol, Pyracinamid, Streptomycin, Prothionamid oder Dapson handeln, wobei die Verwendung eines Aminopenicillins, wie Ampicillin, Amoxycillin, Makrolid oder von Streptomycin besonders bevorzugt ist.

Gemäß einer Ausführungsform der vorliegenden Erfindung wird als Wirkstoff ein Aminopenicillin eingesetzt, das unmittelbar über die Amino-Gruppe des Aminopenicillins kovalent an die Hydroxyethylstärke gekoppelt ist.

Gemäß einer weiteren Ausführungsform wird statt des Aminopenicillin ein Amino.cephalosporin eingesetzt, wodurch eine verringerte Allergenität erreicht wird. Als weitere Ausführungsformen kommen Makrolid-HAS Kopplungen infrage, dabei handelt es sich um Erythromycin oder ein Derivat davon, insbesondere Erythromycylamin. Alternativ dazu kann Streptomycin als Wirkstoff verwendet werden.

Gemäß einer besonders bevorzugten Ausführungsform der vorliegenden Erfindung erfolgt die Kopplung zwischen dem antiinfektiven Wirkstoff und der Hydroxyethylstärke über die reduzierenden Endgruppen der Hydroxyethylstärke.

Gemäß einer weiteren Ausführungsform der vorliegenden Erfindung ist der antiinfektive Wirkstoff über einen Linker an die Hydroxyethylstärke gekoppelt.

Die vorliegende Erfindung umfaßt ferner pharmazeutische Zusammensetzungen, welche eine erfindungsgemäße Verbindung aufweisen. Üblicherweise enthalten die pharmazeutischen Zusammensetzungen ferner einen pharmazeutisch verträglichen Träger.

Schließlich betrifft die vorliegende Erfindung die Verwendung einer der erfindungsgemäßen Verbindungen zur Herstellung eines Arzneimittels zur Behandlung einer Infektionskrankheit. Die pharmazeutische Zusammensetzung ist in besonderem Maße zur Behandlung von Infektionserkrankungen geeignet, welche u.a. durch intrazelluläre Erreger verursacht werden. Dabei kann es sich um das gesamte Spektrum pathogener und fakultativ pathogener Erreger, z.B. bakterielle, virale oder parasitäre Erreger, Mykoplasmen, Mycobakterien, Chlamydien, Rickettsien, etc handeln.

In einem weiteren Aspekt der vorliegenden Erfindung werden HAS-Nukleinsäure-Konjugate zur Verfügung gestellt. Derzeit werden Nukleinsäure-Bibliotheken in großem Umfang auf Nukleinsäuren durchsucht, die eine bestimmte Aktivität aufweisen. Eine entsprechende Aktivität kann beispielsweise die Fähigkeit der Nukleinsäure sein, an bestimmte andere Nukleinsäuren, Rezeptoren oder virale Proteine zu binden. Durch die Bindung kann ein biologisches Signal stimuliert oder inhibiert werden. Für diesen Zweck werden neben den natürlicherweise vorkommenden D-DNA- und D-RNA-Molekülen auch L-DNA- und L-RNA-Moleküle eingesetzt, die sich von den natürlicherweise vorkommenden Molekülen dadurch unterscheiden, dass sie L-Ribose oder L-Desoxyribose anstelle der entsprechenden D-Form als Bestandteile der Nukleinsäuren aufweisen (vgl. WO 98/08856). Im Rahmen der vorliegenden Erfindung konnte gezeigt werden, dass HAS-Nukleinsäure-Konjugate hergestellt werden können, welche die natürliche Funktionalität behalten (vgl. Beispiel 7).

Die vorliegende Erfindung stellt ferner Verfahren zur Herstellung kovalenter HAS-Wirkstoff-Konjugate zur Verfügung. Die Verfahren können in einem wässrigen oder organischen Reaktionsmedium durchgeführt werden, wobei jedoch die Durchführung der Kopplung in der wässrigen Phase bevorzugt ist.

Demgemäß werden Verfahren zur Herstellung eines kovalenten HAS-Wirkstoff-Konjugates zur Verfügung gestellt, bei denen man HAS und mindestens einen Wirkstoff in einem Reaktionsmedium miteinander umsetzt. Das Reaktionsmedium ist dadurch gekennzeichnet, dass es Wasser oder ein Gemisch von Wasser mit organischem Lösungsmittel, welches mindestens 10 Gew.-% Wasser umfaßt, ist.

Das Reaktionsmedium des erfindungsgemäßen Verfahrens umfaßt mindestens 10 Gew.-%, bevorzugt mindestens 50 Gew.-%, insbesondere mindestens 80 Gew.-%, wie beispielsweise 90 Gew.-%, oder sogar bis zu 100 Gew.-%, Wasser, und dementsprechend höchstens 90% Gew.-%, bevorzugt höchstens 50 % Gew.-%, insbesondere höchstens 20 Gew.-%, beispielsweise 10 Gew.-%, oder sogar bis zu 0 Gew.-%, organisches Lösungsmittel. Die Reaktion findet also in einer wäßrigen Phase statt. Das bevorzugte Reaktionsmedium ist Wasser.

Das erfindungsgemäße Verfahren ist schon deshalb von Vorteil, weil nicht zwangsläufig toxikologisch bedenkliches Lösungsmittel eingesetzt werden muss und demzufolge bei dem erfindungsgemäß hergestellten Produkt die Entfernung, auch geringer Reste toxikologisch bedenklicher Lösungsmittel entfällt, wie sie nach dem bekannten Verfahren immer zwingend ist, um die unerwünschte Verunreinigung mit Lösungsmittel zu vermeiden. Weiterhin kann die nach dem bisherigen Verfahren notwendige zusätzliche Qualitätskontrolle auf Reste toxikologisch bedenklicher Lösungsmittel unterbleiben, weil das erfindungsgemäße Verfahren die Verwendung von toxikologisch nicht bedenklichen Lösungsmitteln bevorzugt. Erfindungsgemäss bevorzugte Lösungmittel sind beispielsweise toxikologisch unbedenkliche protische Lösungsmittel wie Ethanol oder Propylenglykol.

Weiterhin ist ein Vorteil des erfindungsgemässen Verfahrens, dass durch organische Lösungsmittel induzierte irreversible oder reversible Strukturänderungen von Proteinen oder Peptiden grundsätzlich vermieden werden, die bei Verfahren in organischen Lösungsmitteln nicht systematisch ausgeschlossen werden können. Die nach den erfindungsgemässen Verfahren erhaltenen Polypeptide können demzufolge von den in DMSO hergestellten verschieden sein.

Erfindungsgemäß wurde ferner überraschenderweise festgestellt, dass eine Kopplung von HAS an Wirkstoffe in einer wässrigen Lösung vorgenommen werden kann, ohne dass dabei in nennenswertem Umfang Nebenreaktionen zu beobachten sind. Das erfindungsgemäße Verfahren führt somit unmittelbar zu verbesserten Produkten in großer Reinheit. Das erfindungsgemäße Verfahren ermöglicht somit erstmals die einfache Herstellung von HAS-Wirkstoff-Konjugaten, in denen der Wirkstoff in aktiver Form vorliegt und die vorteilhaften Eigenschaften der HAS erhalten bleiben. Zur Isolierung des HAS-Wirkstoff-Konjugates aus dem Reaktionsgemisch sind keine besonderen Verfahren erforderlich, da die Reaktion in der wässrigen Phase erfolgt, also nicht zwangsläufig organische Lösungsmittel abgereinigt werden müssen.

Erfindungsgemäß ist es bevorzugt, dass HAS unmittelbar an eine ∈-NH₂-Gruppe, α-NH₂-Gruppe, SH-Gruppe, COOH-Gruppe oder -C(NH₂)₂- Gruppe des Wirkstoffes bindet. Alternativ dazu kann eine weitere reaktive Gruppe in HAS eingeführt werden oder die Bindung zwischen HAS und dem Wirkstoff über einen Linker erfolgen. Die Verwendung von entsprechenden Linkern zur Bindung von Wirkstoffen an PEG ist im Stand der Technik bekannt. Die Verwendung von Aminosäuren, insbesondere Glycin, Alanin, Leucin, Isoleucin, und Phenylalanin, sowie von Hydrazin- und Oxylamin-Derivaten als Linker, wie in WO 97/38727 und EP 605 963 offenbart, ist bevorzugt.

Gemäß einer Ausführungsform des Verfahrens der vorliegenden Erfindung wird HAS vor Bindung an den Wirkstoff oxidiert. Die Oxidation kann nach einem der im Stand der Technik bekannten Verfahren erfolgen, wobei eine selektive Oxidation der reduzierenden Endgruppen der HAS bevorzugt ist. Dieses Vorgehen ermöglicht Verfahren, bei denen die oxidierte reduzierende Endgruppe der HAS mit einer Aminogruppe des Wirkstoffes unter Ausbildung eines Amids reagiert. Diese Ausführungsform weist den besonderen Vorteil auf, dass eine spezifische Bindung zwischen HAS und den Wirkstoffen und somit ein besonders homogenes Produkt erzielt wird.

Dabei läßt man HAS mit oxidierten reduzierenden Endgruppen und den Wirkstoff vorzugsweise mindestens 12, besonders bevorzugt mindestens 24 Stunden miteinander reagieren. Ferner kann es wünschenswert sein, einen beliebigen Aktivator, beispielsweise Ethyldimethyl-aminopropyl-Carbodiimid (EDC), zuzugeben. Das Mol-Verhältnis zwischen HAS und Wirkstoff während der Reaktion kann beliebig gewählt werden, liegt üblicherweise im Bereich von HAS:Wirkstoff von 20:1 bis 1:20, wobei ein Verhältnis von 6:1 bis 1:6 besonders bevorzugt ist. Die besten Ergebnisse wurden bei einem Mol-Verhältnis von HAS:Wirkstoff von etwa 2:1 erzielt.

Andere Kopplungsreaktionen zwischen einer Aminogruppe des Wirkstoffes und HAS sind selbstverständlich auch vom Umfang der Erfindung umfaßt. Beispielsweise Verfahren, bei denen HAS und der Wirkstoff unmittelbar miteinander umgesetzt werden, wobei eine Schiff'sche Base zwischen HAS und Wirkstoff als Zwischenprodukt entsteht. Die Azomethingruppe -CH=N- der Schiff'schen Base kann anschließend unter formaler Addition von <2H> zu einer Methylenamingruppe -CH₂-NH- reduziert werden. Für diese Reduktion kann der Fachmann eine Vielzahl von im Stand der Technik bekannten Reduktionsmitteln verwenden, wobei die Reduktion mittels BH₄ besonders bevorzugt ist.

Die Kopplung des HAS kann an eine beliebige Gruppe des Wirkstoffes erfolgen. Die Kopplung wird vorzugsweise so vorgenommen, dass das metabolisierte Konjugat mindestens 50%, vorzugsweise mindestens 75% der Aktivität des Wirkstoffes vor der Kopplung aufweist, wobei ein Erhalt von mindestens 95% der Aktivität besonders bevorzugt ist. Die Kopplungsreaktion kann natürlich auch so gesteuert werden, dass die Bindung des HAS ausschließlich an eine oder meherere bestimmte Gruppen des Wirkstoffes erfolgt, beispielsweise an Lysin- oder oder Cystein-Reste eines Peptids. Besondere Vorteile ergeben sich, wenn man HAS über die oxidierten reduzierenden Endgruppen an eine oder mehrere bestimmte Gruppen des Wirkstoffes bindet, da bei einem entsprechenden Vorgehen homogene HAS-Wirkstoff-Konjugate erhalten werden.

Gemäß einer bevorzugten Ausführungsform des Verfahrens der vorliegenden Erfindung werden als Ausgangsstoffe für die Reaktion HAS und ein Protein oder ein Peptid eingesetzt. Dabei können beliebige therapeutische oder diagnostische Proteine natürlichen oder rekombinanten Ursprungs verwendet werden. Eine Liste der derzeit auf dem Markt befindlichen Wirkstoffe rekombinanter Herstellung findet sich in Pharma Business, July/August 2000, Seiten 45 bis 60. Die vorliegende Erfindung umfaßt die Herstellung von HAS-Wirkstoff-Konjugaten, die irgend einen der in dieser Veröffentlichung genannten Wirkstoffe umfassen.

Die Herstellung von Konjugaten unter Verwendung von Zytokinen, beispielsweise Interferonen, Interleukinen, Wachstumsfaktoren, Enzymen, Enzym-Inhibitoren, Rezeptoren, Rezeptor-Fragmenten, Insulin, Faktor VIII, Faktor IX, Antibiotika (oder Antiinfektiva), Peptid-Antibiotika, viralen Hüll-Proteinen, Hämoglobinen, Erythropoietin, Albuminen, hTPA, Antikörpern, Antikörperfragmenten, Einzelketten-Antikörpern, DNA, RNA oder ein Derivat derselben ist besonders bevorzugt. Besondere Vorteile ergeben sich bei der Verwendung von rekombinanten Proteinen oder Peptiden in dem erfindungsgemäßen Verfahren. Wie bereits ausgeführt, können entsprechende Proteine häufig aufgrund ihrer für Menschen antigenen Eigenschaften nicht als Wirkstoffe eingesetzt werden. Nach Kopplung an HAS durch die erfindungsgemäßen Verfahren verringert sich jedoch die Immunogenität der rekombinanten Proteine, was den medizinische Einsatz am Menschen ermöglicht.

Besondere Vorteile ergeben sich ferner bei der Kopplung von HAS an kurzkettige Proteine und kleinere Peptide. Derzeit werden eine Vielzahl von Peptidbanken erstellt, beispielsweise Phagen-Display-Banken, bei denen kurze Oligopeptide (beispielsweise 3- bis 20-mere) auf der Oberfläche von Phagen exprimiert werden. Ferner werden Antikörper aus einer einzigen Polypeptidkette (sogenannte "single chain antibodies", Einzelketten-Antikörper) in Bakterien oder auf der Oberfläche von Phagen exprimiert. Diese Banken werden auf bestimmte Wirkstoff- oder Bindungs-Aktivität hin durchgesehen. Die therapeutische und diagnostische Verwendung entsprechender Peptid-Wirkstoffe oder Antikörper scheitert jedoch bislang daran, dass diese im Organismus aufgrund ihrer geringen Größe sehr schnell ausgeschieden werden (vgl. Chapman et al., 1999, a.a.O.) . Nach dem erfindungsgemäßen Verfahren können diese Peptide vorteilhaft an HAS gekoppelt werden, und weisen eine in vivo Halbwertszeit auf, die eine Verwendung als Wirkstoff ermöglicht.

Alternativ zu den obigen Ausführungsformen kann als Wirkstoff ein Hormon, ein Steroidhormon, ein von Aminosäuren abgeleitetes Hormon oder ein von Fettsäuren abgeleitetes Hormon eingesetzt werden. Im Einzelfall mag es notwendig sein, eine aktive Gruppe, beispielsweise mittels Linker, in das Hormon vor der Bindung an HAS einzuführen.

Erfindungsgemäß kann beliebige physiologisch verträgliche HES als Ausgangsmaterial verwendet werden. HES mit einem mittleren Molekulargewicht (Gewichtsmittel) von 1 bis 300 kDa, insbesondere von 1 bis 150 kDa ist bevorzugt. HES mit einem mittleren Molekulargewicht von 2 bis 40 kD ist besonders bevorzugt. HES weist vorzugsweise einen molaren Substitutionsgrad von 0,1 bis 0,8 und ein Verhältnis von C₂ : C₆-Substitution im Bereich von 2 bis 20, jeweils bezogen auf die Hydroxyethylgruppen, auf.

Die Erfindung betrifft ferner die nach den obigen Verfahren erhältlichen HAS-Wirkstoff-Konjugate. Diese Konjugate weisen vorteilhafte Eigenschaften auf, nämlich hohe Aktivität des Wirkstoffs, geringe Immunogenität, lange Verweilzeit im Körper und exzellente rheologische Eigenschaften, welche den medizinischen Nutzen der Konjugate steigern.

Demgemäß umfaßt die vorliegende Erfindung ebenfalls Verfahren zur Herstellung eines Arznei- oder Diagnosemittels, bei denen man ein HAS-Wirkstoff-Konjugat nach einem der obigen Verfahren herstellt und mit einem der im Stand der Technik bekannten, pharmazeutisch verträglichen Träger, Adjuvans oder Hilfsstoff vermischt, sowie nach diesem Verfahren erhältliche Arznei- oder Diagnosemittel.

Die medizinische Verwendung des entsprechenden Arzneimittels hängt von der Art des Wirkstoffs ab. Wird beispielsweise Hämoglobin als Wirkstoff eingesetzt, kann das Konjugat als Sauerstoff-Transport-Mittel verwendet werden. Wird andererseits ein Zytokin als Wirkstoff für die Herstellung verwendet, kann das Konjugat beispielsweise in der Tumortherapie verwendet werden. Die Konzentration des jeweils zu verwendenden Konjugates in dem Arzneimittel kann von jedem durchschnittlichen Fachmann ohne weiteres in Dosis-Wirkungstests ermittelt werden.

Die Diagnosemittel können in vivo oder in vitro zur Diagnose von Erkrankungen oder Störungen verwendet werden. Wenn als Wirkstoff ein Antikörper oder Antikörperfragment eingesetzt wird, eignet sich das Konjugat beispielsweise für die Durchführung der im Stand der Technik üblichen ELISA-Nachweisverfahren.

In den Beispielen wurden die nachfolgend beschriebenen Materialien verwendet:

| | |
|---|---|
| Humanes Serumalbumin: | Sigma-Aldrich A3782 |
| HES 130kD: | Typ 130/0,5, hergestellt aus T91SEP (Fresenius Kabi) |
| Daten: | M_{W}: 130 000 ± 20 OOOD |
| Mₙ: | 42 600 D |
| HES 10 kD: | Typ HHH 4-2 (Fresenius Kabi) |
| Daten: | M_{W}: 9 800 D |
| Mₙ: | 3 695 D |
| EDC: (Ethyldimethyl-aminopropyl-Carbodiimid) | Sigma-Aldrich Nr. 16.146-2 |
| HOBt (1-Hydroxy-1H-Benzotriazolhydrat) | Sigma-Aldrich Nr. 15.726-0 |
| DIG-Glykandetektionskit: | Roche-Boehringer Nr. 1142 372 |

Die folgenden Beispiele 1 bis 6 beschreiben die Kopplung von HSA und Diaminobutan an HES mit oxidierten reduzierenden Endgruppen oder die direkte Kopplung von HSA an HES. HSA und Diaminobutan sind dabei lediglich Beispiele für die oben definierten Wirkstoffe. Beispiel 7 beschreibt die Kopplung von Oligonukleotiden an HES.

### Beispiel 1: Selektive Oxidierung der reduzierenden Endgruppen der Hydroxyethylstärke:

Für die selektive Oxidierung der reduzierenden Endgruppen der Hydroxyethylstärke (130 kD und 10 kD) wurde diese in einer minimalen Menge Wasser aufgelöst und mit einer verschiedenen Menge einer Iod-Lösung und einer KOH-Lösung versetzt.

Die Mischung wurde gerührt, bis die auf I₂ hinweisende Farbe verschwand. Diese Vorgehensweise wurde mehrfach wiederholt, um die Zugabe einer größeren Menge der Iod-Lösung und KOH-Lösung zu erreichen. Die Lösung wurde anschließend über einen Amberlite IR 120 Na⁺ Kationenaustauscherharz gereinigt, für 20 Stunden gegen destilliertes Wasser dialysiert (Dialyseröhrchen mit einer Ausschlussgrenze von 4-6 kD) und lyophylisiert.

Der Grad der Oxidation wurde jeweils mittels des in Somogyi, N. (Method in Carbohydride Chemistry, Vol. 1, (1962) S. 384-386) offenbarten Verfahrens ermittelt. Die Protokolle der Oxidationsreaktion sind in Tabelle 1 wiedergegeben.

**Tabelle 1: Oxidation der reduzierenden Endgruppen der HES (130 kD und 10 kD) unter verschieden Bedingungen**

| Verfahren | HES (Mn) | Iod-Lösg. 0.1N | KOH-Lösg. 0.1N | Lösungsmittel | Reaktionszeit | Ertrag |
|---|---|---|---|---|---|---|
| OXIDATION (1) HES 130 | 1g 2.4x10⁻⁵mol | 0.3 ml 3.0x10⁻⁵mol | 0.5 ml 5.0x10⁻⁵ mol | Wasser 4.0 ml | 4 h 25°C | 30.1% |
| OXIDATION (2) HES 130 | 4g 9.4x10⁻⁵mol | 1.0 ml 1.0x10⁻⁴mol | 1.5 ml 1.5x10⁻⁴ mol | Wasser 6.0 ml | 0. Nacht 25°C | 24.8% |
| OXIDATION (3) HES 130 | 5g 1.2x10⁻⁴mol | 1.2 ml 1.2x10⁻⁴mol | 1.5 ml 1.5x10⁻⁴ mol | Wasser 7.5 ml | 0. Nacht 25°C | 24.3% |
| OXIDATION (4) HES 130 | 5g 1.2x10⁻⁴mol | 3.0 ml 3.0x10⁻⁴mol | 4.5 ml 4.5x10⁻⁴mol | Wasser 7.5 ml | 0. Nacht 25°C | 60.8% |
| OXIDATION (5) HES 130 | 5g 1.2×10⁻⁴mol | 4.0 ml 4.0×10⁻⁴mol | 5 ml 5.0×10⁻⁴ mol | Wasser 7.5 ml | 0. Nacht 25°C | 80.0% |
| OXIDATION (6) HES 130 | 8 g 1.9x10⁻⁴mol | 7.0 ml 7.0×10⁻⁴mol | 11.5 ml 1.2x10⁻³ mol | Wasser 7.5 ml | 0. Nacht 25°C | 88,4% |
| OXIDATION (7) HES 130 | 10 g 2.4x10⁻⁴mol | 10 ml 1.0x10⁻³mol | 20 ml 2.0x10⁻³ mol | Wasser 7.5 ml | 0. Nacht 25°C | 100% |
| OXIDATION (1) HES 10 | 5 g 1.4x10⁻³mol | 2.0 ml 2.0x10⁻⁴mol | 2.0 ml 2.0x10⁻⁴ mol | Wasser 10.0 ml | 20 h 25°C | 3.0% |
| OXIDATION (2) HES 10 | 5g 1.4x10⁻³mol | 3.5 ml 3.5x10⁻⁴mol | 4.5 ml 4.5x10⁻⁴ mol | Wasser 10.0 ml | 0. Nacht 25°C | 5.3% |
| OXIDATION (3) HES 10 | 15 g 4.1x10⁻³mol | 21.0 ml 2.1x10⁻³mol | 31.0 ml 3.1x10⁻³ mol | Wasser | 0. Nacht 25°C | 10.5% |
| OXIDATION (4) HES 10 | 8 g 2.2x10⁻³mol | 83.0 ml 8.3x10⁻³mol | 180.0 ml 1.8x10⁻² mol | Wasser | 0. Nacht 25°C | 80.0% |
| OXIDATION (5) HES 10 | 7 g 1.9x10⁻³mol | 95.0 ml 9.5x10⁻³mol | 210.0 ml 2.1x10⁻² mol | Wasser | 0. Nacht 25°C | 100.0% |
| OXIDATION (6) HES 10 | 6.4 g 1.7x10⁻³mol | 50 ml 5.0x10⁻³ | 150 ml 1.5x10⁻² | Wasser | ü. Nacht 25°C | 100.0% |

Die in dieser Tabelle zusammengefaßten Protokolle werden nachfolgend für die Oxidation (6), HES 10 kD, im Detail wiedergegeben: 6.4 g HES 10kD (1.7 mmol) wurden in einem Reaktionsgefäß unter ständigem Rühren in wenigen ml Wasser gelöst. Dazu wurden 50 ml einer 0.1 N Jodlösung (5.0 mmol) und 150 ml einer 0.1 N KOH-Lösung (15 mmol) gegeben. Diese Mischung wurde über Nacht bei 25°C stehen gelassen. Der Mix wurde mit Amberlite IR 120 (Na+-Form) gereinigt und gegen Wasser dialysiert (Dialyseschlauch Celluloseacetat; Cut-off 4 bis 6 kD). Das dialysierte Produkt wurde lyophilisiert (Heraeus-Christ Alpha, Kolbentrocknung über Nacht).

Wie Tabelle 1 zu entnehmen ist, wurde eine vollständige Oxidation der reduzierenden Endgruppen (entspricht Ertrag 100%) der HES 130 kD erzielt, nachdem die Iod-Menge von 3,0 x 10⁻⁵ mol bis auf 1,0 × 10⁻³ mol erhöht wurde.

Für eine vollständige Oxidation der reduzierenden Endgruppen der HES 10 kD war eine weitere Steigerung der Tod-Menge auf eine Konzentration von mehr als 2,1 x 10⁻³ mol notwendig.

### Beispiel 2: Bindung von HES mit oxidierten reduzierenden Endgruppen an HSA in wässriger Phase:

Für die Kopplung wurde Hydroxyethylstärke mit oxidierten reduzierenden Endgruppen (ox-HES) und HSA vollständig in Wasser gelöst. Als die Lösung klar war, wurde Ethyldimethyl-aminopropyl-Carbodiimid (EDC) in Wasser gelöst zugegeben. Nach Aktivierung durch EDC bei gemäßigtem Rühren wurden weitere Mengen an EDC zugegeben. Die Reaktion wurde gegebenenfalls mit HOBt aktiviert und über Nacht stehengelassen. Das Produkt wurde durch Dialyse gegen destilliertes Wasser für 15 Stunden gereinigt und anschließend lyophylisiert (nachfolgend als Verfahren A bezeichnet).

Die Protokolle der Kopplungsreaktion finden sich in Tabelle 2.

**Tabelle 2: Kopplungsreaktionen zwischen HES (130 kD und 10 kD) mit oxidierten reduzierenden Endgruppen und HSA unter verschiedenen Bedingungen (Verfahren A; Zahl in der Klammer in Spalte HES gibt das Oxidationsverfahren nach Tabelle 1 wieder)**

| Verfahren A | HSA | ox-HES (Mn) | EDC | HOBt | Lösungsmittel | Aktivier. | Reaktion |
|---|---|---|---|---|---|---|---|
| Kopplung I ox-HES 130 | 300 mg 4.4x10⁻⁶inol | 100 mg (1) 2.4x10⁻⁶mol | 25 mg 1.6x10⁻⁴mol | 100 mg 7.7x10⁻⁴mol | H₂0/Dioxan 13 ml/2 ml | 1.5 h 3-4°C | 4 h 25°C |
| Kopplung II ox-HES 130 | 100 mg 1.5x10⁻⁶mol | 300 mg (2) 7.0x10⁻⁶mol | 15 mg 9.7x10⁻⁵mol | 100 mg 7.7x10⁻⁴mol | H₂0/Dioxan 10 ml/3ml | 1.5 h 3-4°C | 0. Nacht 25°C |
| KopplungIII ox-HES 130 | 200 mg 3.0x10⁻⁶mol | 3.8 g (5) 8.9x10⁻⁵mol | 46.5 mg 3.0x10⁻⁵ mol | 20 mg 1.5x10⁻⁴mol | H₂0/Dioxan 10 ml/3 ml | 0 h | 24°h 25°C |
| Kopplung IV ox-HES 130 | 100 mg 1.5x10⁻⁶mol | 1.9 g (5) 4.5x10⁻⁵mol | 25 mg 1.6x10⁻⁴mol | 20 mg 1.5x10⁻⁴mo | Wasser | 1.5 h 3-4°C | ü. Nacht 25°C |
| Kopplung V ox-HES 130 | 200 mg 3.0x10⁻⁶mol | 4.3 g (5) 1.0x10⁻⁴ mol | 100 mg 6.0x10⁻⁴ mol | 0 mg | Wasser | 0 h | ü. Nacht 25°C |
| Kopplung VI ox-HES 130 | 100 mg 1.5x10⁻⁶ | 130 mg (7) 3.0x10⁻⁶ mol | 50 mg 3.0x10⁻⁴ mol | 0 mg | Wasser* 5ml + 10ml | 0 h | 5 h 25°C |
| KopplungVII ox-HES 10 | 100 mg 1,5x10⁻⁶ | 130 mg (7) 3.0x10⁻⁶mol | 200 mg 3.0x10⁻⁴ mol | 0 mg | Wasser* 5ml + 2x 10ml | 0 h | 24 h 25°C |
| Kopplung 1 ox-HES 10 | 100 mg 1.5x10⁻⁶ mol | 300 mg (1) 8.1x10⁻⁵mol | 5 mg 3.0x10⁻⁵ mol | 100 mg 7.7x10⁻⁴mol | H₂0/Dioxan 13 ml/2 ml | 1.5 h 3-4°C | 0. Nacht 25°C |
| Kopplung 11 ox-HES 10 | 70 mg 1.0x10⁻⁶mol | 1.0 g (2) 2.7x10⁻⁴ mol | 15.5 mg 1.0x10 mol | 0 mg | Wasser | 10 ml 0 h | 0. Nacht 25°C |
| KopplungIII ox-HES 10 | 200 mg 3.0x10⁻⁶mol | 3.0 g (3) 8.1x10⁻⁴mol | 77.5 mg 5.0x10⁻⁴mol | 20 mg 1.5x10⁻⁴mol | Wasser | 0 h | 6 h 25°C |
| Kopplung IV ox-HES 10 | 50 mg 7.4x10⁻⁷mol | 7.4 g (4) 2.0x10⁻³mol | 282 mg 1.5x10⁻³mol | 0 mg | Wasser | 0 h | 0. Nacht 25°C |
| Kopplung V ox-HES 10 | 100 mg 1.5x10⁻⁶mol | 103 g (6) 2.8x10 mol | 93 mg 5.6x10⁻⁴mol | 0 mg | Wasser* 4 ml | 0 h | 20 h 25°C |
| Kopplung VI ox-HES 10 | 100 mg 1.5x10⁻⁶mol | 103 g (6) 2.8x10 mol | 200 mg 1.2x10⁻³mol | 0 mg | Wasser 3x5 ml | 0 h | 30 h 25 °C |

| | | | | | | | |
|---|---|---|---|---|---|---|---|
| - Zugabe der EDC-Lösung mit einem Tropftrichter innerhalb von 60 Min. | | | | | | | |

Die Kopplungsreaktion VII zwischen ox-HES 130 kD und HSA wird im folgenden im Detail erläutet: In einem Rundkolben wurden 130 mg ox-HES 130 kD (Oxidationsgrad ca. 100%) und 100 mg HSA unter Rühren in ca. 5 ml Wasser bei Raumtemperatur gelöst. Sobald die Lösung klar war, wurden 200 mg EDC in 3 Portionen jeweils in 5-10 ml Wasser gelöst über einen Zeitraum von einer Stunde zugetropft. Zwischen jeder Zugabe ließ man ca. 4 h bei Raumtemperatur rühren. Nach 24 h Reaktionszeit wurde das Gemisch gegen Wasser dialysiert (Dialyseschlauch Celluloseacetat; Cutoff 4-6 kD). Anschließend wurde das Produkt lyophilisiert.

### Beispiel 3: Direkte Bindung von HES an HSA in wässriger Phase:

Das Prinzip dieser Reaktion basiert auf der Bildung von Schiff'schen Basen zwischen HES und Aminogruppen des Proteins, wobei die Reaktion durch die Reaktion der Schiff'schen Base zu dem entsprechenden Amin mittels NaBH₄ gesteuert wird (nachfolgend als Verfahren B bezeichnet).

Dafür wurde HES vollständig in wenig Wasser gelöst. Dazu wurde in Boratpuffer, pH 9.0, gelöstes HSA gegeben. Zu dieser Lösung wurde NaBH₄ gegeben und bei Raumtemperatur unter Rühren belassen. Ein weiteres Aliquot von HES 130 kD, gefolgt von weiterem NaBH₄ wurden zugegeben. Nach Abschluß der Reaktion wurde wie beschrieben dialysiert und gefriergetrocknet.

Die Protokolle der einzelnen Versuche sind in Tabelle 3 zusammengefaßt.

**Tabelle 3: Direkte Kopplung zwischen HES (130 kD und 10 kD) und HSA unter verschiedenen Bedingungen (Verfahren B)**

| Verfahren B | HSA | HES (Mn) | NaBH4 | Puffer PH | Reaktionszeit |
|---|---|---|---|---|---|
| Kopplung I | 50 mg | 500 mg | 500 mg | Na₂HPO₄. 0 ml | 48 h |
| HES 130 | 7.5x10⁻⁷ mol | 1.2x10⁻⁵mol | 1.3x10⁻²mol | 7.4 | 25°C |
| Kopplung II | 100 mg | 1.0 g | 60 mg | Na₂HPO₄, 1 ml | 20 h |
| HES 130 | 1.5x10⁻⁶mol | 2.4x10⁻⁵mol | 1.6x10⁻³mol | 7.4 | 25°C |
| Kopplung III | 50 mg | 9.8 g | 285 mg | Na₂HP0₄. 1 ml | 36 h |
| HES 130 | 7.5x10⁻⁷mol | 2.3x10⁻⁴mol | 7.5x10⁻³mol | 7.4 | 25°C |
| Kopplung IV | 50 mg | 2.0 g | 180 mg | Borat 0.1M | 30 h |
| HES 130 | 7.5x10⁻⁷mol | 4,7x10⁻⁵mol | 4.7x10⁻³mol | 9.0 | 25°C |
| Kopplung V | 50 mg | 4.0 g | 60 mg | Borat 0.1M | 100 h |
| HES 130 | 7.5x10⁻⁷mol | 9.4x10⁻⁵mol | 1.6x10⁻³mol | 9.0 | 25 °C |
| Kopplung I | 50 mg | 2.8 g | 28 mg | Borat 0.1M | 80 h |
| HES 10 | 7.5x10⁻⁷ mol | 9.4x10⁻⁵mol | 1.6x10⁻³ mol | 9.0 | 25°C |

Im Einzelnen wurden für die Kopplung der HES 130 kD 2.0 g dieser Verbindung vollständig in Wasser gelöst (ca. 5 ml). Dazu wurden 50 mg in 1 ml 0.1 M Boratpuffer, pH 9,0, gelöstes HSA gegeben. Zu dieser Lösung wurden 30 mg NaBH₄ gegeben und unter Rühren bei Raumtemperatur belassen. Nach 18 h wurde ein weiteres Aliquot von 2.0 g HES 130 kD, gefolgt von weiteren 30 mg NaBH₄ zugegeben. Nach insgesamt 100 h Reaktionszeit wurde dialysiert und gefriergetrocknet (Kopplung V, HES 130 kD).

Für die Kopplung der HES 10 kD wurden 1.4 g dieser Verbindung komplett in Wasser gelöst (ca. 5 ml). Dazu wurden 50 mg in 1 ml 0,1 M Boratpuffer, pH 9,0, gelöstes HSA gegeben. Zu dieser Lösung wurden 14 mg NaBH₄ gegeben und unter Rühren bei Raumtemperatur belassen. Nach 18 h wurde ein weiteres Aliquot von 1.4 g HES 10 kD, gefolgt von weiteren 14 mg NaBH₄ zugegeben. Nach insgesamt 80 h Reaktionszeit wurde dialyisiert und gefriergetrocknet (Kopplung I, HES 10 kD).

### Beispiel 4 Analyse der Kopplungsprodukte mittels GPC:

Die Reaktionsprodukte wurden zunächst unter Verwendung von Gel-Permeations-Chromatographie (GPC) analysiert.

4.1 Für die GPC wurde ein FPLC-Gerät (Pharmacia), welches mit einem HPLC UV Monitor (Hitachi) verbunden war, verwendet. Ferner wurden die folgenden Bedingungen verwendet:

| | |
|---|---|
| Säule: | Superose 12 HR 10/30 (1×30 cm) (Pharmacia) |
| UV-Monitor: | 280 nm |
| Pumpe: | 0,2 ml/min |
| Puffer: | 50 mM Phosphat/150 mM NaCl pH 7,2. |

Unter diesen Bedingungen wird der HSA-Peak üblicherweise nach 63 min gefunden, wobei zusätzlich ein kleiner Peak bei etwa 57 min gemessen werden kann, der durch HSA-Dimere verursacht wird. Die mittels GPC erhaltenen Chromatogramme lassen sich wie folgt analysieren:

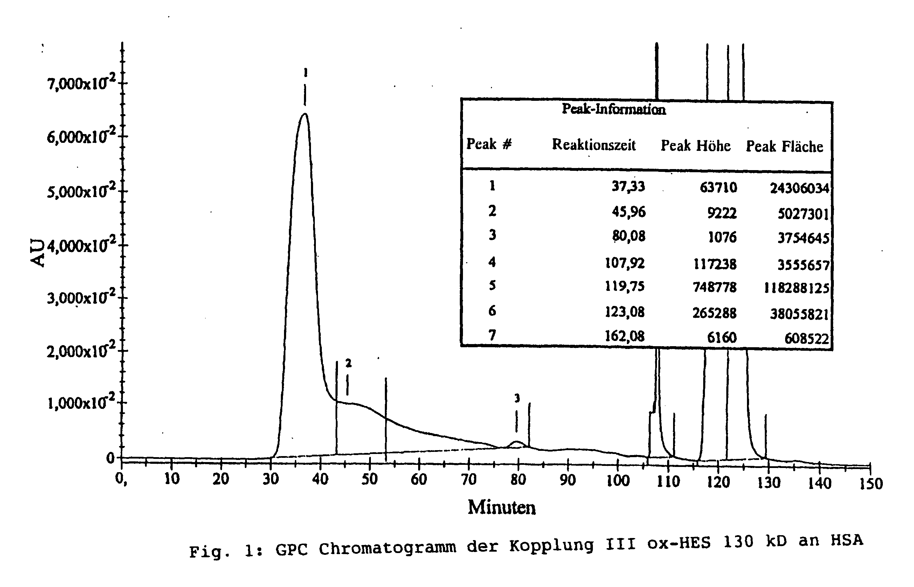

4.2 Fig. 1 ist ein Chromatogramm, das die Größenverteilung der Produkte nach Kopplung von ox-HES 130 kD an HSA (Kopplung III) zeigt. Bei diesem Kopplungsverfahren wurden ohne HOBt-Aktivierung sehr gute Ergebnisse erzielt. Ein deutlicher, einzelner breiter Peak wurde bei 37 Min. und eine weitere, kleinere Bande bei 45 Min. gemessen, was auf ein Kopplungsprodukt mit höherem Molekulargewicht als HSA hinweist. Gleichzeitig wurden Spuren von nicht-modifiziertem HSA gefunden.

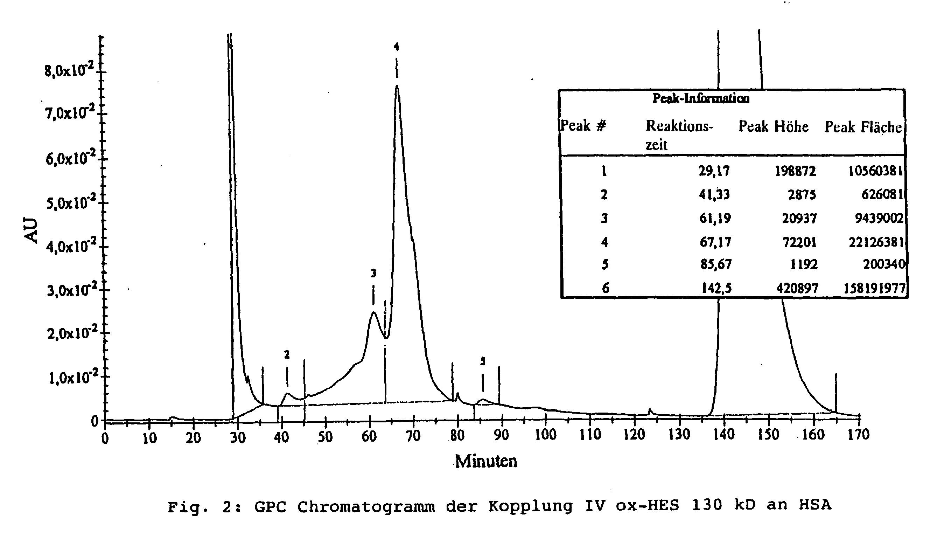

Fig. 2 zeigt die Größenverteilung der Produkte nach Kopplung von ox-HES 130 kD an HSA (Kopplung IV). Die Reaktion wurde mit HOBt aktiviert. Es zeigt sich, dass diese Aktivierung die Ausbeute verringert, möglicherweise durch Förderung von Nebenreaktionen.

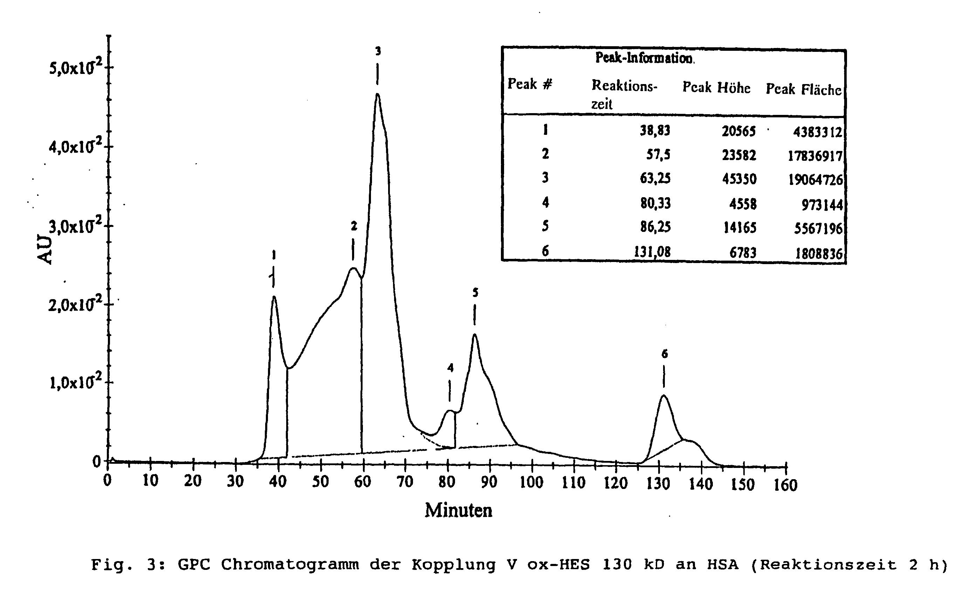

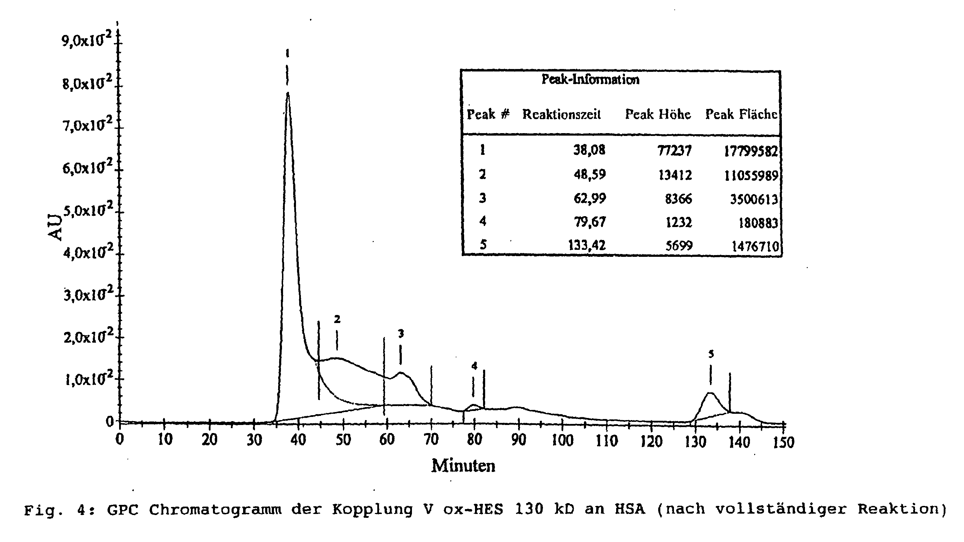

Die Fig. 3 und 4 zeigen die Größenverteilung der Reaktionsprodukte während und nach der Kopplungsreaktion von ox-HES 130 kD an HSA (Kopplung V) . Nach 2 Stunden Reaktionszeit wurde nicht-modifiziertes HSA als Produkt mit der höchsten Konzentration, daneben aber auch erste Kopplungsprodukte mit höherem Molekulargewicht gefunden. Nach Ablauf der Reaktion wurde ein homogenes Kopplungsprodukt mit einer Retentionszeit von ca. 35 Min. in hoher Konzentration gefunden. Nicht-modifiziertes HSA und andere Kopplungsprodukte lagen in relativ geringer Konzentration vor.

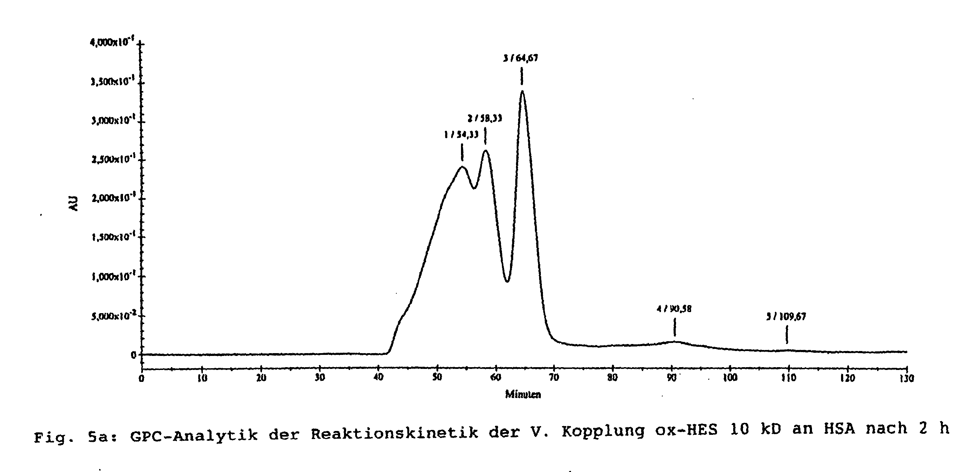

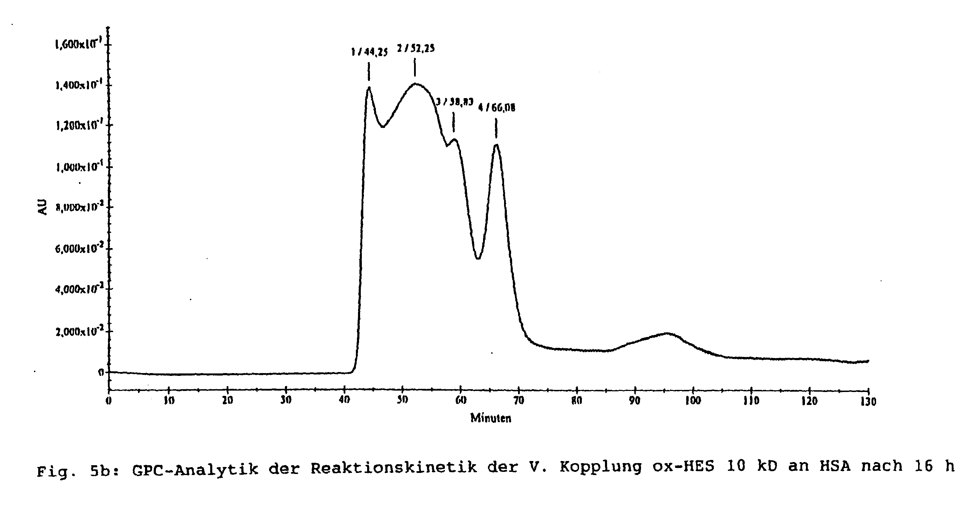

Fig. 5 zeigt die entsprechende Größenverteilung der Reaktionsprodukte während und nach der Kopplungsreaktion von ox-HES 10 kD an HSA (Kopplung V). Auch hier zeigt sich, dass die Konzentration der Kopplungsprodukte mit einem Molekulargewicht, das oberhalb des Gewichtes von HSA liegt, im Verlauf der Reaktion zunimmt.

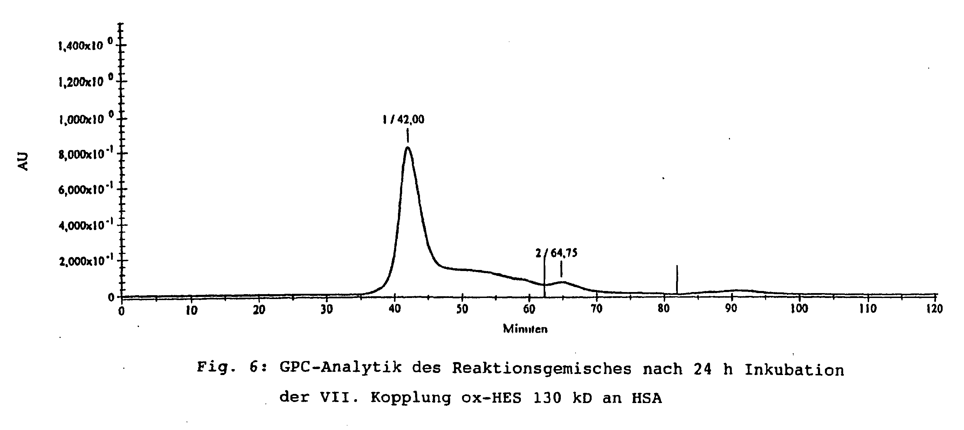

Eine Kopplungsreaktion, bei der nahezu alle HSA-Moleküle in ein homogenes Kopplungsprodukt überführt werden konnten, wird schließlich in Fig. 6 gezeigt (Reaktionsprodukte der Kopplung VII).

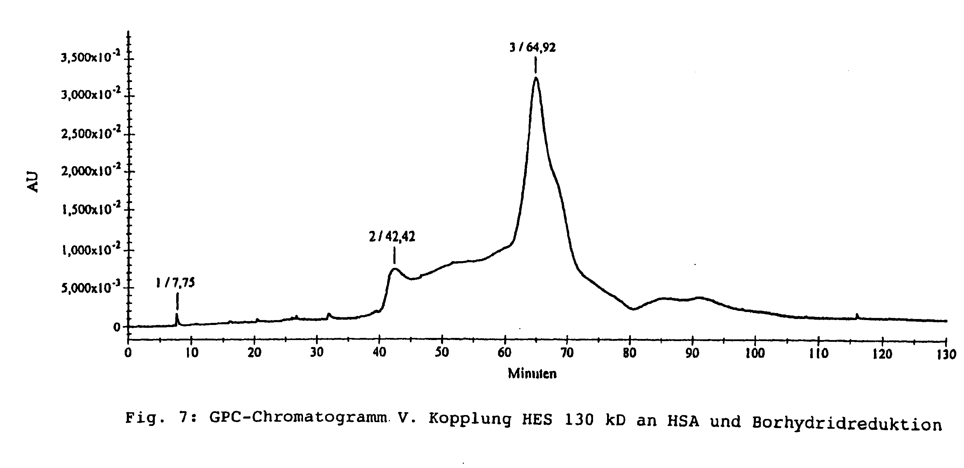

4.2 Ein Beispiel für die Chromatogramme, die bei der Analyse der direkten Kopplung von HES an HSA erhalten wurden, wird in Fig. 7 gezeigt (Verfahren B, HES 130 kD, Kopplung V). Ein deutlicher Peak bei ca. 65 Min. (HSA) ist zu erkennen. Darüber hinaus wurde aber auch ein Kopplungsprodukt nachgewiesen (Peak bei ca. 42 Min.).

### Beispiel 5 Analyse der Kopplungsprodukte mittels SDS-PAGE und Western Blot:

5.1 PAGE wurde in Gegenwart von Natriumdodecylsulfat (SDS) unter Verwendung eines Miniprotean II Gerätes der Firma Biorad und 7,5 % Acrylamidgele durchgeführt. Die Durchführung der Elektrophorese erfolgte nach den Vorschriften des Herstellers. Die Gele wurden nach dem Verfahren von Blum (Elektrophoresis, Vol. 8, (1997) S. 93-99) mit Silber gefärbt, um Proteine sichtbar zu machen.

Die Gegenwart von Glykanen in den Kopplungsprodukten wurde mittels Western-Blot und Glykan-Detektions-Kit der Firma Roche-Boehringer nachgewiesen. Nach Auf trennung der Produkte mittels SDS-PAGE wurden diese unter Verwendung des Blotting-Gerätes der Miniprotean II Elektrophoreseeinheit auf eine Nitrocellulosemembran überführt. Die Membran wurde anschließend mittels Periodat unter Bedingungen oxidiert, bei denen lediglich die vicinalen OH-Gruppe oxidiert werden. Diese wurden anschließend mit einem Amino-funktionalisierten Digoxygenin umgesetzt. Gebundenes Digoxygenin wurde mittels spezifischer monoklonaler Antikörper, welche an eine alkaline Phosphatase gebunden waren, nachgewiesen. Dafür wurde ein Substrat der Phosphatase (4-Nitro-tetrazoliumchlorid/5-Brom-4-chlor-3-indolylphosphat) zugegeben, welches ein schwerlösliches blau-violettes Produkt erzeugt. Dieses Produkt schlägt sich auf der Membran nieder und lässt somit die Banden sichtbar werden. Nicht-modifiziertes HSA und Kreatinase wurden als Negativkontrollen eingesetzt, während Transferrin als Positivkontrolle fungierte.

Die genauen Verfahrensschritte sind im Beipackzettel zu diesem Kit beschrieben (Roche-Boehringer).

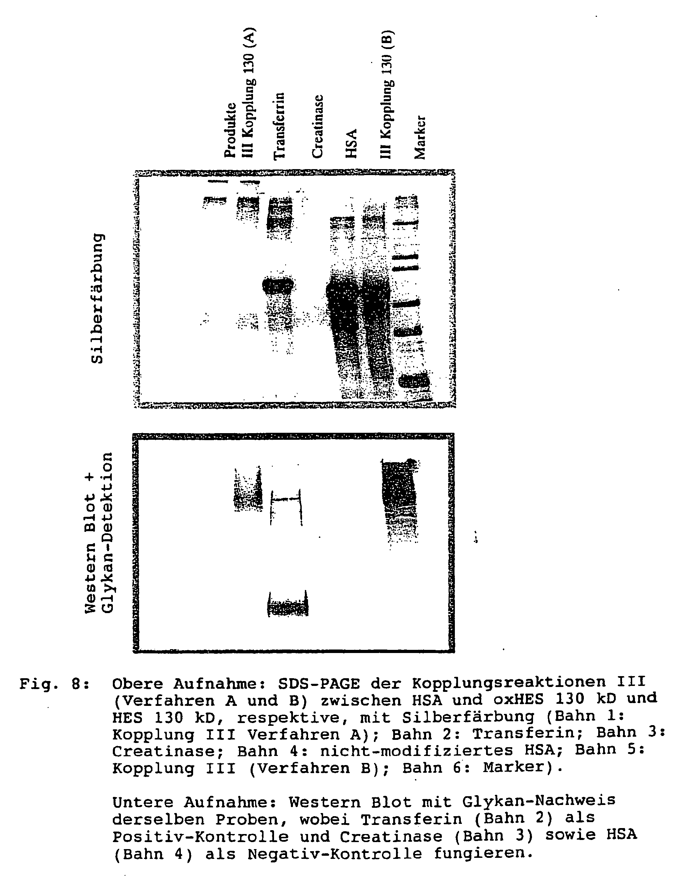

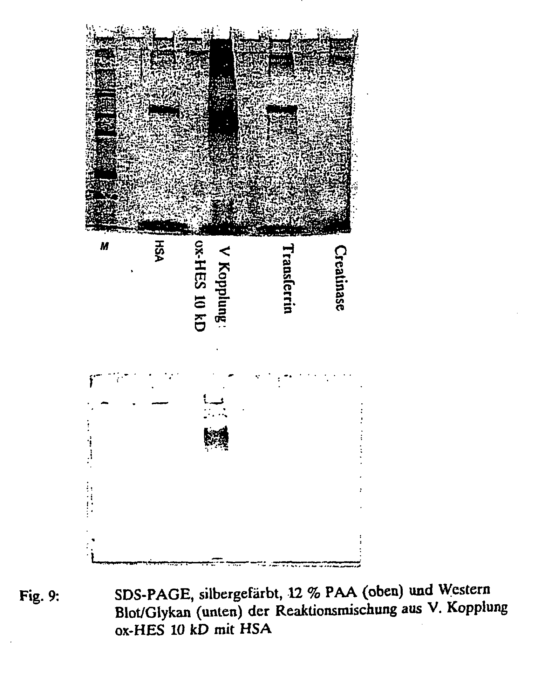

5.2 Die Fig. 8 und 9 zeigen jeweils eine Aufnahme des Silbergefärbten SDS-PAGE-Gels (oben) und die Aufnahme der entsprechenden Produkte nach Übertragung auf eine Membran und Glykannachweis (unten). Wie sich aus diesen Figuren entnehmen läßt, entsteht während der Kopplungsreaktion ein relativ homogenes Glykan als Reaktionsprodukt, während gleichzeitig die Konzentration an nicht-modifiziertem HSA abnimmt.

### Beispiel 6 Abklärung möglicher Nebenreaktionen:

Zur Klärung, ob Nebenreaktionen in Form einer Selbstkondensation von HES mit oxidierten reduzierenden Endgruppen erfolgen, wurden folgende Reaktionsansätze erstellt:

**Tabelle 4: Reaktionsansätze zur Abklärung von Nebenreaktionen**

| | ox-HES | EDC | HOBt | Wasser | Reaktionszeit |
|---|---|---|---|---|---|
| HES 130 kD | 500 mg 1.2x10⁻⁵ mol | 15 mg 7.8x10⁻⁵ mol | - | 5.0ml | 30 h 25 °C |
| HES 130 kD | 500 mg 1.2x10⁻⁵ mol | 15 mg 7.8x10⁻⁵ mol | gesättigte Lösung | 5.0 ml | 30 h 25°C |
| HES 10 kD | 100 mg 2.7x10⁻⁵mol | 3.4 mg 1.8x10⁻⁵ mol | - | 5.0 ml | 30 h 25°C |
| HES 10 kD | 100 mg 2.7x10⁻⁵ mol | 3.4 mg 1.8x10⁻⁵ mol | gesättigte Lösung | 5.0 ml | 30 h 25°C |
| HES 130 kD | 700 mg 1.6x10⁻⁵mol | 31 mg 1.6x10⁻⁴mol | - | 5.0 ml | 30 h 25 °C |
| HES 130 kD | 700 mg 1.6x10⁻⁵mol | 31 mg 1.6x10⁻⁴mol | gesättigte Lösung | 5.0 ml | 30 h 25°C |

Ziel der Experimente war es, nachzuweisen, inwieweit eine mögliche Selbstkondensation von HES in Gegenwart oder Abwesenheit von HOBt stattfindet. Die Proben wurden lyophilisiert und analysiert.

Mittels GPC und Streulichtmessungen konnten innerhalb der Detektionsgrenzen von einigen Prozent keine Hinweise auf Molekulargewichtsvergrößerungen gefunden werden.

### Beispiel 7: Kopplung von oxidierter HES an DNA und Analyse der Funktionalität der Kopplungsprodukte

### Reaktionsprinzip:

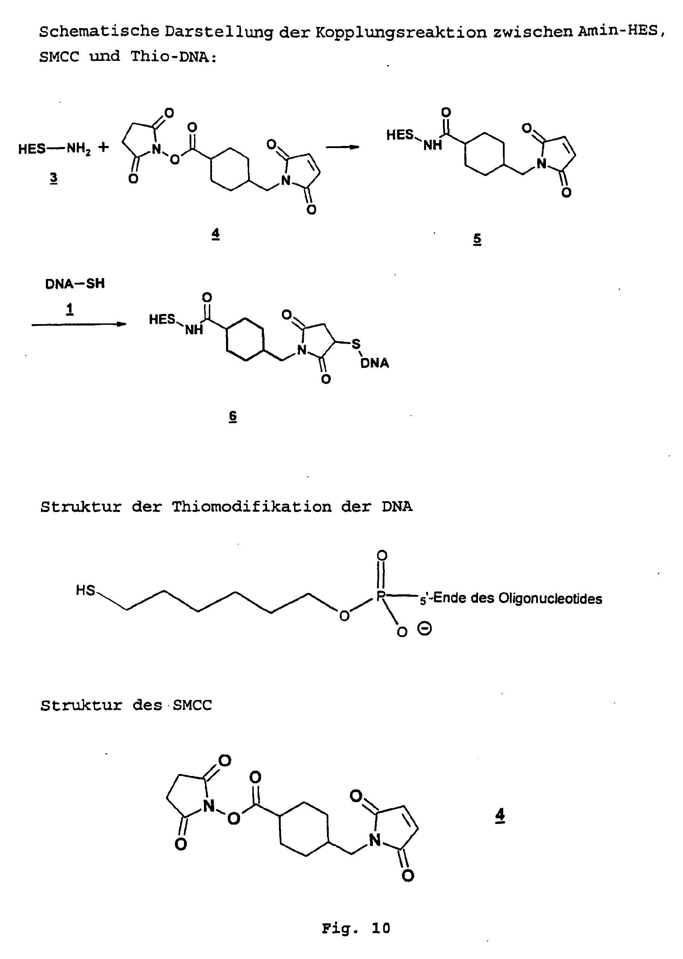

Eine schematische Darstellung der Reaktion findet sich in Fig. 10. Im ersten Schritt reagiert die Aminogruppe der Amin-HES12KD 3 mit der N-Hydroxysuccinimid-gruppe des SMCC 4 zum Konjugat 5. Durchs Zentrifugieren mit dem Zentrifugen-Dialyseeinsatz wird nicht reagiertes SMCC 4 abgetrennt. Die Maleimid-Gruppe des Konjugats 5 reagiert dann mit der Thiol-Gruppe der Thio-DNA 1 zum dem gewünschten Produkt 6. Der in 6 fett markierte Bereich ist nur ein Spacer und kann jede Gestalt haben.

Die Überprüfung der biologischen Aktivität des Konjugates 6 erfolgte über die Spaltung durch das Restriktionsenzym EcoR1. Ein Restriktionsenzym schneidet nur doppelsträngige DNA mit intakter Erkennungssequenz.

### DNA:

Verwendet wurde doppelsträngige DNA, synthetisiert von MWG Biotech AG, Ebersberg. Die Sequenzen der Einzelstränge lauten: 
SEQ ID NO. 1: 5' - GTAGAGACAGGAGGCAGCAGTTGAATTCGCAGGGT-
GAGTAGCAGTAGAGC-3' ;
 
SEQ ID NO. 2: 5' - GCTCTACTGCTACTCACCCTGCGAATT-
CAACTGCTGCCTCCTGTCTCTAC-3';
 modifiziert mit 5'Thiol C6 S-S durch MWG (vgl. Fig. 10).

Die beiden DNA Einzelstränge wurden in bidest. Wasser in einer Konzentration von je 2 µg/µl gelöst und im Verhältnis 1 : 1 bei 96°C zu einer doppelsträngigen Thio-DNA 1 mit einer Konzentration von 2 µg/µL hybridisiert.

### Analyse der Produkte:

Die Analyse erfolgte durch Gelelektrophorese in einem 4% Agarose-Gel mit einem TBE-Laufpuffer aus 45 mM Tris-Borat, 1mM EDTA, pH 8.0 von je 1 µg DNA in Gegenwart von 50 µg Ethidiumbromid pro 100 ml Gel. Die Fotos wurden aufgenommen mit einem CCD-System Modular (INTAS Imaging Instruments, Göttingen, D) und einem UV-Transilluminator UVT-20 S/M/L (Herolab GmbH, Wiesloch, D) bei 312 nm.

Vom Reaktionsgemisch wurden 1 µL (1µg DNA) abgenommen und geschnitten mit 1 µL (20 U) EcoR1- Restriktionsenzym (New England Biolabs GmbH, Schwalbach/Taunus, D), 1 µL Reaktionspuffer (50 mM Natriumchlorid, 100 mM Tris-HCl, 10 mM Magnesiumchlorid, 0,025% Triton X-100, pH 7.5 von New England Biolabs) und 7µL bidest.
Wasser bei 37°C für 3h.

### Modifikation der HES

Die Oxidation von HES12KD (Fresenius, Charge 2540SR2.5P) mit einem mittleren Molgewicht von 12000 g/mol mit Iod-Lösung zum Oxo-HES12KD 2 wurde nach den in DE 19628705 offenbarten Verfahren durchgeführt.

### Reaktion von 1,4-Diaminobutan mit Oxo-HES12KD 2:

1,44 g (0,12 mmol) des Oxo-HES12KD 2 wurden in ml wasserfreiem Dimethylsulfoxid (DMSO) gelöst, unter Stickstoff zu einer Lösung aus 1.5 ml (1,50 mmol) -1,4 Diaminobutan getropft und bei 40°C für 19 h gerührt. Das Reaktionsgemisch wurde zu einem Gemisch aus 80 ml Ethanol und 80 ml Aceton gegeben. Der gebildete Niederschlag wurde abzentrifugiert und in 40 ml Wasser aufgenommen. Die Lösung wurde für 4 Tage gegen Wasser dialysiert (SnakeSkin Dialyseschlauch, 3.5 KD cut off, Perbio Science Deutschland GmbH, Bonn, D) und anschließend gefriergetrocknet. Die Ausbeute betrug 80% (1.06 g) Amino-HES12KD 3.

### Kopplung von Amino-HES12KD 3. an Thio-DNA 1.

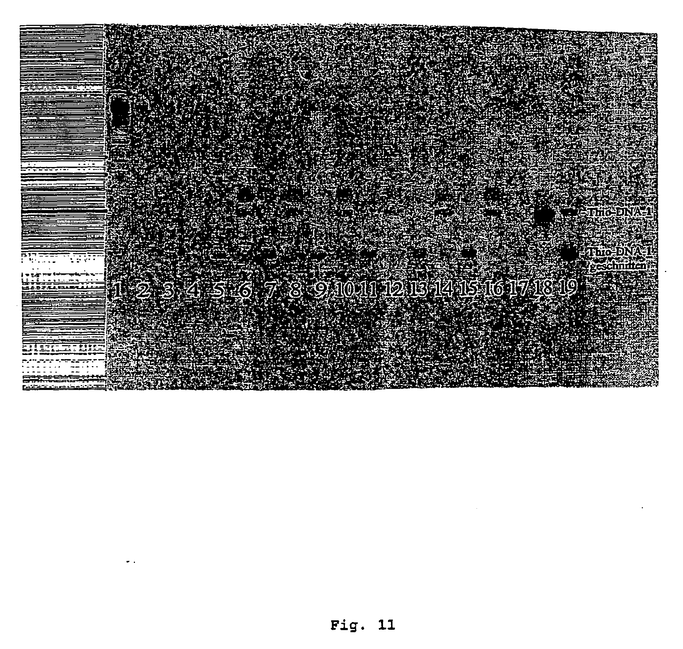

Zu 400 *µ*L einer 10 mg/mL-Lösung aus Amino-HES12KD **3**. in einem Puffer aus 10 mM Natriumphosphat und 150 mM Natriumchlorid, pH 7.44, wurden 1 mg SMCC 4 gelöst in 50 µL wasserfreiem DMSO gegeben und das Gemisch 80 min bei Raumtemperatur und 10 min bei 46°C behandelt. Dann wurde das Gemisch abzentifugiert, der Überstand vom Niederschlag abgenommen und erneut zentrifugiert. Vom Überstand wurden 200 µl abgenommen und 45 min bei 14000 g mit einem MICROCON YM-3 (Amicon, Millipore GmbH, Eschborn, D) Zentrifugen-Dialyseeinsatz zentrifugiert. Nach Zugabe von 400 µL Puffer aus 10 mM Natriumphosphat und 150 mM Natiurmchlorid, pH 7.44 wurde erneut für 45 min zentrifugiert, weitere 400 µL Puffer zugegeben und 60 min zentrifugiert. Die im Dialyseeinsatz verbliebene Menge an Konjugatlösung wurde auf 50 µL aufgefüllt. Von dieser Lösung wurden 10 µL zu 10 µL der Thio-DNA-Lösung. 1 gegeben und 14 h bei Raumtemperatur reagieren lassen. Zur Analyse wurden 1 µL abgenommen. Die Ergebnisse zeigt Fig. 11 in Bahn 2 und 3.

Die Reaktionsbedingungen für den beschriebenen Versuch und für Versuche mit abgewandelten Reaktionsbedingungen sind in der Tabelle 1 zusammengefasst und die Ergebnisse im Fig. 11 dargestellt.

### Zusammenfassung der Ergebnisse:

Es wurden folgende Bedingungen untersucht:
1. Menge an SMCC: 1 mg (Bahnen 2, 6, 10, 14) bzw. 5.6 mg (Bahnen 4, 8, 12, 16);
2. Temperatur für die Reaktion mit der Thio-DNA 1:
   Raumtemperatur (Bahnen 2, 4, 6, 8) bzw. 37°C (Bahnen 10, 12, 14, 16);
3. Pufferbedingungen:
   10 mM Phosphat, 150 mM NaCl ohne EDTA, pH 7.44 (Bahnen 2, 4, 10, 12) bzw;
   100 mM Phosphat, 150 mM NaCl + 50 mM EDTA, pH 7,23 (Bahnen 6, 8, 14, 16)

In Figur 11, Bahnen 2-18 sind die Ergebnisse der 8 Kopplungsversuchen von Amino-HES12KD 3 an die Thio-DNA 1 mit Hilfe von SMCC dargestellt. Dabei sind die Ergebnisse direkt aus der Reaktion bzw. nach der Reaktion und anschließendem Schneiden der DNA nebeneinander dargestellt. In Bahn 1 ist ein Gemisch verschiedener Referenz-DNAs als Längenmarker aufgetragen und die Bahnen 18 und 19 zeigen Thio-DNA 1 bzw. geschnittene Thio-DNA 1. Alle Versuche zeigen zusätzlich zu noch nicht umgesetzter Thio-DNA 1 Kopplungsprodukte bei höheren Massen (Bahnen 2, 4, 6, 8, 10, 12, 14, 16). Da HES12KD ein Gemisch unterschiedlich großer Moleküle ist, zeigen auch die Kopplungsprodukte eine Molgewichtsverteilung. Alle Köpplungsprodukte enthalten intakte DNA, da sie von EcoR1 vollständig verdaut werden können. Dies zeigt sich im fast vollständigen Verschwinden der entsprechenden diffusen Banden nach dem Schneiden (Bahnen 3, 5, 7, 9, 11, 13, 15, 17).

**Tabelle 1:Reaktionsbedingungen der Kopplung von Amino-HES12KD 3 an die Thio-DNA 1**

| **Bahn** | **Versuch** | **Temp [°C]** | **SMCC[mg]** | **DNA** | **HES12KD** | **Puffer** | |
|---|---|---|---|---|---|---|---|
| 1 | Marker | | | | | | |
| 2 | 19A1 | RT | 1 | Thio-DNA | Amino-HES12KD | 7.44, 10mM | |
| 3 | | | | - | | | geschnitten |
| 4 | 19B1 | RT | 5.6 | Thio-DNA | Amino-HES12KD | 7.44, 10mM | |
| 5 | | | | | | | geschnitten |
| 6 | 19C1 | RT | 1 | Thio-DNA | Amino-HES12KD | 7.23, 100mM | |
| 7 | | | | | | | geschnitten |
| 8 | 19D1 | RT | 5.6 | Thio-DNA | Amino-HES12KD | 7.23, 100mM | |
| 9 | | | | | | | geschnitten |
| 10 | 19A2 | 37°C | 1 | Thio-DNA | Amino-HES12KD | 7.44, 10mM | |
| 11 | | | | | | | geschnitten |
| 12 | 19B2 | 37°C | 5.6 | Thio-DNA | Amino-HES12KD | 7.44, 10mM | |
| 13 | | | | | | | geschnitten |
| 14 | 19C2 | 37°C | 1 | Thio-DNA | Amino-HES12KD | 7.23, 100mM | |
| 15 | | | | | | | geschnitten |
| 16 | 19D2 | 37°C | 5.6 | Thio-DNA | Amino-HES12KD | 7.23, 100mM | |
| 17 | | | | | | | geschnitten |
| 18 | | | | Thio-DNA | | | |
| 19 | | | | | | | geschnitten |

## Patentansprüche

1. HAS-Nukleinsäure-Konjugat, erhältlich durch ein Verfahren zur Herstellung eines kovalenten HAS-Nukleinsäure-Konjugates, bei dem man HAS und eine Nukleinsäure in einem Reaktionsmedium miteinander umsetzt, **dadurch gekennzeichnet, dass** das Reaktionsmedium Wasser oder ein Gemisch von Wasser mit einem organischen Lösungsmittel, welches mindestens 10 Gew.-% Wasser umfasst, ist.

2. Konjugat nach Anspruch 1, wobei die Nukleinsäure eine D- oder L-Nukleinsäure ist, insbesondere eine D-DNA, L-DNA, D-RNA oder L-RNA.

3. HAS-Wirkstoff-Konjugat, erhältlich durch Verfahren zur Herstellung eines kovalenten HAS-Wirkstoff-Konjugates, bei dem man HAS und einen Wirkstoff in einem Reaktionsmedium miteinander umsetzt, **dadurch gekennzeichnet, dass** das Reaktionsmedium Wasser oder ein Gemisch von Wasser mit organischem Lösungsmittel, welches mindestens 10 Gew.-% Wasser umfasst, ist.

4. Verbindung, die ein Konjugat aus einer HAS und einem Wirkstoff umfasst, wobei die HAS entweder unmittelbar oder über einen Linker kovalent an den Wirkstoff gebunden ist.

5. Verbindung gemäß Anspruch 3 oder 4, in welcher der Wirkstoff ein Vitamin, Impfstoff, Toxin, Antibiotikum, Antiarrythmikum, Appetitzügler, Anästhetikum, Analgetikum, Antirheumatikum, Antiallergikum, Antiasthmatikum, Antidepressivum, Antidiabetikum, Antihistaminikum, Antihypertonikum, oder ein antineoplastisches Mittel ist.

6. Verbindung gemäß einem der Ansprüche 3 bis 5, in welcher der Wirkstoff ein Hormon, Steroid, Lipid, Protein, Oligo- oder Polypeptid oder eine Nukleinsäure, insbesondere eine D- oder L-Nukleinsäure, wie beispielsweise eine D-DNA, L-DNA, D-RNA oder L-RNA ist.

7. Verbindung gemäß einem der Ansprüche 3 bis 6, in welcher der Wirkstoff ein Enzym, Enzym-Inhibitor, Rezeptor, Rezeptor-Fragment, Insulin, Faktor VIII, Faktor IX, Zytokin, Interferon, Interleukin, Wachstumsfaktor, Peptid-Antibiotikum, virales Hüll-Protein, Hämoglobin, Erythropoietin, Albumin, hTPA, Antikörper, Antikörperfragment, Einzelketten-Antikörper oder ein Derivat davon ist.

8. Verbindung gemäß einem der Ansprüche 3 bis 7, in welcher der Wirkstoff ein Steroidhormon, ein von Aminosäuren abgeleitetes Hormon oder ein von Fettsäuren abgeleitetes Hormon ist.

9. Verbindung gemäß einem der Ansprüche 3 bis 8, in die HAS an die ε-N-H₂-Gruppe, an die α-NH₂-Gruppe, an die SH Gruppe an eine COOH-Gruppe oder an eine - C(NH₂)₂-Gruppe eines Wirkstoffes bindet.

10. Verbindung gemäß einem der Ansprüche 3 bis 9, in der die HAS Hydroxyethylstärke ist und vorzugsweise ein mittleres Molekulargewicht (Gewichtsmittel) von 1 bis 300 kDa, vorzugsweise von 5 bis 200 kDa, aufweist.

11. Verbindung gemäß einem der Ansprüche 3 bis 10, in der die Hydroxyethylstärke einen molaren Substitutionsgrad von 0,1 bis 0,8 und ein Verhältnis von C₂ : C₆₋Substitution im Bereich von 2 bis 20, jeweils bezogen auf die Hydroxyethylgruppen, aufweist.

12. Verbindung gemäß einem der Ansprüche 3 bis 11, in der der antineoplastische Wirkstoff aus der Gruppe bestehend aus Alkylantien, Antimetaboliten, Antibiotika oder Naturstoffen ausgewählt wurde.

13. Verbindung gemäß einem der Ansprüche 3 bis 12, in der der antineoplastische Wirkstoff Mitomycin C, Cyclophosphamid, Bleomycin, Chlorambucil, Cisplatin, Ara-C, Fludarabin, Doxorubicin, Etoposid, 5-FU, MTX, Vinblastin, Vincristin, Vindesin, Hydroxyurea, 6-MP oder CCNU ist.

14. Verbindung gemäß einem der Ansprüche 3 bis 13, in der der antineoplastische Wirkstoff an die reduzierenden Endgruppen der Hydroxyethylstärke gebunden ist.

15. Verbindung gemäß einem der Ansprüche 3 bis 14, in der der antineoplastische Wirkstoff Mitomycin C ist.

16. Verbindung gemäß einem der Ansprüche 3 bis 15, in der Hydroxyethylstärke an die Methoxy-Gruppe des Mitomycin C gekoppelt ist.

17. Verbindung gemäß einem der Ansprüche 3 bis 16, in der Hydroxyethylstärke über einen Linker an die Methoxy-Gruppe des Mitomycin C gekoppelt ist.

18. Pharmazeutische Zusammensetzung, welche eine Verbindung gemäß einem der Ansprüche 3 bis 17 aufweist.

19. Pharmazeutische Zusammensetzung gemäß Anspruch 18, die ferner einen pharmazeutisch verträglichen Träger aufweist.

20. Pharmazeutische Zusammensetzung gemäß Anspruch 18 oder 19, die zusätzlich ein Zytokin umfasst.

21. Pharmazeutische Zusammensetzung gemäß einem der Ansprüche 18 bis 20, in der das Zytokin IL-2, α-Interferon oder γ-Interferon ist.

22. Verwendung einer Verbindung gemäß einem der Ansprüche 12 bis 17 zur Herstellung eines Arzneimittels zur Behandlung von Tumoren und/oder deren Metastasen.

23. Verwendung gemäß Anspruch 22 zur Herstellung eines Arzneimittels zur Behandlung von urologischen Tumoren und/oder Metastasen urologischer Tumore.

24. Verwendung gemäß Anspruch 23 zur Herstellung eines Arzneimittels wobei das Arzneimittel zur Behandlung des Nierenzellkarzinoms oder zur Behandlung von Metastasen des Nierenzellkarzinoms verwendet wird.

25. Verwendung gemäß Anspruch 24 zur Herstellung eines Arzneimittels zur Behandlung des klarzelligen oder chromophilen Nierenzellkarzinoms oder Metastasen derselben.

26. Verwendung gemäß Anspruch 22, wobei das Arzneimittel zur Behandlung lymphatischer Systemerkrankungen verwendet wird.

27. Verwendung gemäß Anspruch 26, bei der die lymphatische Systemerkrankung CLL, Hodgkin-Lymphom, NHL, multiples Myelom, Morbus Waldenström ist.

28. Verwendung gemäß Anspruch 27, wobei als antineoplastischer Wirkstoff Fludarabin verwendet wird.

29. Verwendung gemäß Anspruch 22, bei der der Tumor ein kutanes/lokales primäres Malignom oder dessen Metastasen ist.

30. Verwendung gemäß Anspruch 29, bei der der Tumor eine hämatologische Systemerkrankung oder eine onkologische Erkrankung ist, beispielsweise ein nichtkleinzelliges und kleinzelliges Bronchialkarzinom, Mammakarzinom, Ösophagus Plattenepithelkarzinom, Nierenzellkarzinom, Hodenkarzinom, malignes Melanom, ALL oder CML ist.

31. Verwendung gemäß einem der Ansprüche 22 bis 30, bei der das Arzneimittel ferner ein Zytokin umfasst.

32. Verwendung gemäß einem der Ansprüche 22 bis 31, bei der das Zytokin IL-2, α-Interferon oder γ-Interferon ist.

33. Verbindung gemäß einem der Ansprüche 3 bis 11, in der der antiarrhythmische Wirkstoff aus der Gruppe bestehend aus Natriumkanal-Blocker, Beta-Rezeptoren-Blocker, selektive Repolarisationsverlängerer, Calcium-Antagonisten und Lokalanästhetika ausgewählt wurde.

34. Verbindung gemäß einem der Ansprüche 3 bis 11, in der der antiarrhythmische Wirkstoff Adenosin, Chinidin, Procainamid, Disopyramid, Lidocain, Phenytoin, Mexiletin, Ajamalin, Parjmalium, Propafenon, Atenolol, Propanolol, Amiodaron, Sotalol, Verapamil, Gallopamil oder Diltiazem ist.

35. Verbindung gemäß einem der Ansprüche 3 bis 11, in der der antiarrhythmische Wirkstoff an die reduzierenden Endgruppen der Hydroxylethylstärke gebunden ist.

36. Verbindung gemäß einem der Ansprüche 3 bis 11, in der der antiarrhythmische Wirkstoff Adenosin ist.

37. Verbindung gemäß einem der Ansprüche 3 bis 11, in der der antiarrhythmische Wirkstoff Adenosin ist, welcher über die Aminogruppe an Hydroxylethylstärke gebunden ist.

38. Verbindung gemäß einem der Ansprüche 3 bis 11, in der der antiarrhythmische Wirkstoff über einen Linker an die Hydroxyethylstärke gekoppelt ist.

39. Pharmazeutische Zusammensetzung, welche eine Verbindung gemäß einem der Ansprüche 33 bis 38 umfasst.

40. Pharmazeutische Zusammensetzung gemäß Anspruch 39, die ferner einen pharmazeutisch verträglichen Träger umfasst.

41. Verwendung einer Verbindung gemäß einem der Ansprüche 33 bis 38 zur Herstellung eines Arzneimittels zur Behandlung von Herzrhythmusstörungen, insbesondere ventrikulären Herzrhythmusstörungen.

42. Verwendung einer Verbindung gemäß einem der Ansprüche 39 bis 41 zur Herstellung eines Arzneimittels zur Induktion von Apoptose, wobei die Apoptose bevorzugt in Tumorgewebe induziert wird.

43. Verbindung gemäß einem der Ansprüche 3 bis 11, in der der Wirkstoff ein antiinfektiver Wirkstoff beziehungsweise ein Antibiotikum ist, vorzugsweise aus der Gruppe bestehend aus Aminopenicilline, Cephalosporine und Aminocephalosporine, Beta-Laktam-Antibiotika, Carbapeneme, Aminoglycoside, Tetracycline, Makrolid-Antibiotika, Gyrasehemmer, Glycopeptid-Antibiotika, Lincomycine, Streptogramine, Eveminomicine, Oxazolidinone, Nitroimidazole, Sulfonamide, Cotrimoxazol, Lokalantibiotika, Virustatika, Antimycotika, Tuberculostatika, ausgewählt.

44. Verbindung gemäß einem der Ansprüche 3 bis 11, in der der antiinfektive Wirkstoff Ampicillin, Amoxicillin, Cefotaxim, Ceftazidim, Vancomycin, Clindamycin, Metronidazol, Isoniazid, Rifampicin, Rifabutin, Rifapentin, Ethambutol, Pyracinamid, Streptomycin, Prothionamid oder Dapson ist.

45. Verbindung gemäß einem der Ansprüche 3 bis 11, in der der antiinfektive Wirkstoff ein Aminopenicillin, wie Ampicillin, Amoxycillin, Makrolid oder Streptomycin ist.

46. Verbindung gemäß einem der Ansprüche 3 bis 11, in der der Wirkstoff ein Aminopenicillin ist und die kovalente Kopplung unmittelbar an Hydroxyethylstärke über die Amino-Gruppe des Aminopenicillins erfolgt.

47. Verbindung gemäß einem der Ansprüche 3 bis 11, in der der antiinfektive Wirkstoff Erythromycin oder ein Derivat davon ist.

48. Verbindung gemäß einem der Ansprüche 3 bis 11, in der das Erythromycin-Derivat Erythromicylamin ist

49. Verbindung gemäß einem der Ansprüche 3 bis 11, in der der antiinfektive Wirkstoff Streptomycin ist.

50. Verbindung gemäß einem der Ansprüche 3 bis 11, in der der antiinfektive Wirkstoff an die reduzierenden Endgruppen der Hydroxylethylstärke gebunden ist.

51. Verbindung gemäß einem der Ansprüche 3 bis 11, in der der antiinfektive Wirkstoff über einen Linker an die Hy droxyethylstärke gekoppelt ist.

52. Pharmazeutische Zusammensetzung, welche eine Verbindung gemäß einem der Ansprüche 43 bis 51 aufweist.

53. Pharmazeutische Zusammensetzung gemäß Anspruch 52, die ferner einen pharmazeutisch verträglichen Träger aufweist.

54. Verwendung einer Verbindung gemäß einem der Ansprüche 43 bis 51 zur Herstellung eines Arzneimittels zur Behandlung einer Infektionskrankheit.

55. Verwendung gemäß Anspruch 54, wobei die pharmazeutische Zusammensetzung zur Behandlung von Infektionserkrankungen geeignet ist, welche unter anderem durch intrazelluläre Erreger verursacht werden.

56. Verwendung gemäß Anspruch 54 oder 55, wobei die pharmazeutische Zusammensetzung zur Behandlung von Infektionserkrankungen geeignet ist, welche durch pathogene oder fakultativ pathogene Erreger verursacht werden, z. B. bakterielle, virale oder parasitäre Erreger, Mykoplasmen, Mycobakterien, Chlamydien oder Rickettsien.

57. Verfahren zur Herstellung eines kovalenten HAS-Wirkstoff-Konjugates, bei dem man HAS und einen Wirkstoff in einem Reaktionsmedium miteinander umsetzt, **dadurch gekennzeichnet, dass** das Reaktionsmedium Wasser oder ein Gemisch von Wasser mit organischem Lösungsmittel, welches mindestens 10 Gew.-% Wasser umfasst, ist.

58. Verfahren nach Anspruch 57, bei dem der Wirkstoff ein Vitamin, Impfstoff, Toxin, Antibiotikum (Antiinfektivum), Antiarrhytmikum, Appetitzügler, Anästhetikum, Analgetikum, Antirheumatikum, Antiallergikum, Antiasthmatikum, Antidepressivum, Antidiabetikum, Antihistaminikum, Antihypertonikum, oder ein antineoplastisches Mittel ist.

59. Verfahren nach Anspruch 57 oder 58, bei dem der Wirkstoff ein Hormon, Steroid, Lipid, Protein, Oligo- oder Polypeptid oder eine Nukleinsäure ist.

60. Verfahren nach einem der Ansprüche 57 bis 59, bei dem der Wirkstoff ein Enzym, Enzym-Inhibitor, Rezeptor, Rezeptor-Fragment, Insulin, Faktor VIII, Faktor IX, Zytokin, Interferon, Interleukin, Wachstumsfaktor, Peptid-Antibiotikum, virales Hüll-Protein, Hämoglobin, Erythropoietin, Albumin, hTPA, Antikörper, Antikörperfragment, Einzelketten-Antikörper, eine Nukleinsäure, insbesondere eine D- oder L-Nukleinsäure, wie beispielsweise eine D-DNA, L-DNA, D-RNA oder L-RNA oder ein Derivat davon ist.

61. Verfahren nach einem der Ansprüche 57 bis 60, bei dem der Wirkstoff ein Steroidhormon, ein von Aminosäuren abgeleitetes Hormon oder ein von Fettsäuren abgeleitetes Hormon ist.

62. Verfahren nach einem der Ansprüche 57 bis 61, bei dem man HAS an die ε-NH₂₋Gruppe, an die α-NH₂-Gruppe, an die SH-Gruppe an eine COOH-Gruppe oder an eine -C(NH₂) ₂-Gruppe des Wirkstoffes bindet.

63. Verfahren nach einem der Ansprüche 57 bis 62, bei dem man HAS vor Bindung an den Wirkstoff oxidiert.

64. Verfahren nach Anspruch 63, bei dem man die reduzierende Endgruppe der HAS selektiv oxidiert.

65. Verfahren nach einem der Ansprüche 57 bis 64, bei dem die oxidierte reduzierende Endgruppe HAS mit einer Aminogruppe des Wirkstoffes unter Ausbildung eines Amides reagiert.

66. Verfahren nach einem der Ansprüche 57 bis 65, bei dem man eine Aminogruppe des Wirkstoffes mit HAS so umsetzt, dass eine Schiffsche Base gebildet wird.

67. Verfahren nach Anspruch 66, bei dem die Azomethingruppe -CH=N- der Schiffschen Base zu einer Methylenamingruppe -CH₂-NH- reduziert wird.

68. Verfahren nach einem der Ansprüche 57 bis 67, bei dem als Reduktionsmittel BH₄₋verwendet wird.

69. Verfahren nach einem der Ansprüche 57 bis 68, bei dem man den Wirkstoff oder HAS vor der Herstellung des Konjugates an einen Linker bindet.

70. Verfahren nach einem der Ansprüche 57 bis 68, bei dem man Hydroxyethylstärke mit einem mittleren Molekulargewicht (Gewichtsmittel) von 1 bis 300 kDa verwendet.

71. Verfahren nach einem der Ansprüche 57 bis 70, bei dem man Hydroxyethylstärke mit einem mittleren Molekulargewicht von 2 bis 40 kDa verwendet.

72. Verfahren nach einem der Ansprüche 57 bis 71, bei dem man Hydroxyethylstärke mit einem molaren Substitutionsgrad von 0,1 bis 0,8 und ein Verhältnis von C₂ : C₆₋Substitution im Bereich von 2 bis 20, jeweils bezogen auf die Hydroxyethylgruppen, verwendet.

73. Verfahren zur Herstellung eines Arznei- oder Diagnosemittels, umfassend Schritte bei denen man ein HAS-Wirkstoff-Konjugat nach einem der Ansprüche 57 bis 72 herstellt und mit einem pharmazeutisch verträglichen Träger, Adjuvans oder Hilfsstoff vermischt.

74. Arzneimittel, erhältlich nach Anspruch 73.
